# EUROPEAN PATENT APPLICATION

(11) **EP 4 458 954 A1**
(43) Date of publication of application: **06.11.2024**
(21) Application number: 22916047.8
(22) Date of filing: 26.12.2022
(51) Int. Cl.: C12N 5/079, A61K 35/28, A61P 43/00, C07K 14/78, C12N 5/0775, C12Q 1/6851, C12N 5/10, C12N 15/12

(54) **NEURAL CREST CELL CULTURING METHOD AND PRODUCTION METHOD**

(30) Priority: 27.12.2021 JP 2021212693
(71) Applicant: Sumitomo Pharma Co., Ltd., Osaka-shi, Osaka 541-0045 (JP)
(72) Inventor: TAHARA, Masahiro, Kobe-shi, Hyogo 650-0047 (JP); FUJIKI, Ayaka, Kobe-shi, Hyogo 650-0047 (JP); YOSHIDA, Kenji, Kobe-shi, Hyogo 650-0047 (JP); KITAGAWA, Maiko, Kobe-shi, Hyogo 650-0047 (JP)
(74) Representative: Hoffmann Eitle
(86) International application number: PCT/JP2022/047993
(87) International publication number: WO 2023/127824

(57) **Abstract**

The present invention aims to provide a culturing method and a method for producing a cell population containing a neural crest cell, the methods being able to stably mass produce the neural crest cell from a pluripotent stem cell with quality that allows the cell to be used in a therapy with a cell, and a method for producing a mesenchymal stem cell and the like using the neural crest cell. The method for selectively culturing a neural crest cell in a cell population containing a neural crest cell and/or a neural crest progenitor cell of the present invention comprises a culturing step of adherent culturing the cell population containing the neural crest cell and/or the neural crest progenitor cell, in the presence of one or more extracellular matrices selected from the group consisting of laminin with its α chain being an α1 chain and its β chain being a β1 chain, laminin with its α chain being an α2 chain and its β chain being a β1 chain, laminin with its α chain being an α2 chain and its β chain being a β2 chain, and laminin with its α chain being an α5 chain and its β chain being a β1 chain, and extracellular matrices containing integrin-binding sites of these laminins.

## Description

### Technical Field

The present invention relates to a neural crest cell culturing method and a production method.

### Background Art

A neural crest cell is a cell derived from a neural crest and differentiates into various cell strains such as a neuron of the peripheral nervous system, a Schwann cell, a melanocyte, an endocrine cell, a smooth muscle, a head skeleton, a cornea, and an iris. It is therefore also referred to as the "fourth germ layer" that was acquired during the course of vertebrate evolution.

Recently, there has been proposed a method for inducing differentiation of a pluripotent stem cell such as an iPS cell into a neural crest cell, and further inducing differentiation of the neural crest cell into a mesenchymal stem cell (Patent Literatures 1 to 4, and Non Patent Literatures 1 and 2). Also, in order to purify a desired neural crest cell, there has been proposed a technique for selecting a neural crest cell marker-positive cell by a sorting such as FACS, after inducing differentiation from an iPS cell to the neural crest cell in the course of the method (e.g., Non Patent Literature 1). However, there has been demanded for establishing a method for producing a neural crest cell, which can eliminate contamination during the production processes, and in which the safety is higher, in a clinical application.

On the other hand, integrin, a receptor of extracellular matrix fibronectin and laminin, has been reported to be essential for interaction between the neural crest cell and the extracellular matrix (Non Patent Literature 3). Further, Patent Literature 5 illustrates a method for controlling differentiation into a neural crest cell in the presence of laminin 211 or an E8 fragment of laminin 211. Also, Non Patent Literature 4 reports that when inducing an iPS cell into an eye cell, if using a culturing container coated with laminin 211, the iPS cell is induced into a neural crest cell. Moreover, Patent Literature 6 discloses a method for purifying a neural crest cell, comprising expansion culturing by using laminin 211 as a scaffold for the cell.

For culturing a mesenchymal stem cell, for example, a medium for a mesenchymal stem cell disclosed in Patent Literature 7 is known.

### Citation List

### Patent Literature

Patent Literature 1: WO2011/149762
Patent Literature 2: WO2016/114285
Patent Literature 3: WO2018/199142
Patent Literature 4: WO2019/107485
Patent Literature 5: WO2018/143312
Patent Literature 6: WO2020/230832
Patent Literature 7: WO2016/027850

### Non Patent Literature

Non Patent Literature 1: Makoto Fukuta et al., "Derivation of Mesenchymal Stromal Cells from Pluripotent Stem Cells through a Neural Crest Lineage using Small Molecule Compounds with Defined Media", PLoS One. 2014 Dec 2; 9(12):e112291
Non Patent Literature 2: Alan W. Leung et al., "WNT/β -catenin signaling mediates human neural crest induction via a pre-neural border intermediate", Development, 143:398-410, 2016
Non Patent Literature 3: Laura S. Gammill et al., "Division of labor during trunk neural crest development", Dev Biol. 2010 Aug 15; 344(2): 555-565
Non Patent Literature 4: Shun Shibata et al., "Selective Laminin-Directed Differentiation of Human Induced Pluripotent Stem Cells into Distinct Ocular Lineages", Cell Reports, 2018, 25(6):1668-1679

### Summary of Invention

### Technical Problem

To appropriately sort or select and proliferate desired cells in a therapy with a cell, and to eliminate a risk of occurrence of contamination such as a bacterium during a culturing step are significant problems to be solved.

The present invention aims to provide a method for culturing a cell population containing a neural crest cell and a method for producing the same, the methods being able to stably mass produce the neural crest cell from a pluripotent stem cell with a quality that allows the cell to be used in a therapy with a cell, and a method for producing a mesenchymal stem cell and the like using the neural crest cell.

In addition, since the neural crest cell can differentiate into various cells, another object of the present invention is to select neural crest cells to obtain a neural crest cell of desired cell conditions so that the neural crest cell has a characteristic that can differentiate into desired cells.

### Solution to Problem

The present inventors have found that culturing a cell population containing a neural crest cell and/or a neural crest progenitor cell that are differentiation-induced from a pluripotent stem cell, using a specific laminin or a fragment thereof as a scaffold can result in obtaining a cell population containing a neural crest cell of high purity, and therefore, accomplished a method for stably and efficiently producing a neural crest cell having a quality desirable for the clinical application.

That is, the present invention relates to the following respective inventions.
[1] A method for selectively culturing a neural crest cell in a cell population comprising a neural crest cell and/or a neural crest progenitor cell, the method comprising a culturing step of adherent culturing the cell population comprising the neural crest cell and/or the neural crest progenitor cell, in the presence of one or more extracellular matrices selected from the group consisting of a laminin with its α chain being an α1 chain and its β chain being a β1 chain, a laminin with its α chain being an α2 chain and its β chain being a β1 chain, a laminin with its α chain being an α2 chain and its β chain being a β2 chain, and a laminin with its α chain being an α5 chain and its β chain being a β1 chain, and extracellular matrices comprising integrin-binding sites of these laminins.
[2] The culturing method according to [1], wherein the γ chain of the laminin is a γ1 chain.
[3] The culturing method according to [1] or [2], wherein the laminin is laminin 221.
[4] The culturing method according to any of [1] to [3], wherein the integrin-binding site of the laminin is an E8 fragment of the laminin.
[5] The culturing method according to any of [1] to [4], wherein the cell population after culturing in the culturing step expresses at least one gene selected from EDN3, TFAP2A, NGFR and TWIST1.
[6] The culturing method according to any of [1] to [5], wherein at least a part of a culture surface with the cell population adhered thereon is coated with the extracellular matrix, and the cell population is adherent cultured in a medium in the culturing step.
[7] The culturing method according to [6], wherein the medium is a medium suitable for maintaining the neural crest cell.
[8] The culturing method according to any of [1] to [7], wherein both the total number of cells and the proportion of the neural crest cell to the total number of cells in the cell population after culturing increase compared to the cell population before culturing.
[9] A method for producing a cell population comprising a neural crest cell, the method comprising:
   (1) a step of inducing differentiation of a pluripotent stem cell into a neural crest cell and/or a neural crest progenitor cell to obtain a cell population comprising the neural crest cell and/or the neural crest progenitor cell; and
   (2) a step of adherent culturing the cell population obtained in step (1) by the culturing method according to any of [1] to [8].
[10] The production method according to [9], wherein the cell population obtained in step (1) is subjected to step (2) without being sorted or selected with a neural crest cell marker.
[11] The production method according to [9] or [10], wherein step (1) comprises culturing a cell population comprising a pluripotent stem cell in a medium comprising at least one SMAD signaling inhibitor and at least one Wnt signaling activator.
[12] The production method according to [11], wherein the SMAD signaling inhibitor is a TGFβ inhibitor.
[13] The production method according to [11] or [12], wherein the Wnt signaling activator is a GSK3β inhibitor.
[14] The production method according to any of [9] to [13], wherein step (1) is conducted for 7 to 18 days.
[15] The production method according to any of [9] to [14], wherein the medium in step (1) is a chemically defined medium without serum replacement or without serum.
[16] A method for producing a cell population comprising a mesenchymal stem cell, the method comprising:
   (3) a step of producing a cell population comprising a neural crest cell by the production method according to any of [9] to [15]; and
   (4) a step of culturing the cell population obtained in step (3) to induce differentiation into a mesenchymal stem cell in the presence of bFGF.
[17] A cell population comprising a mesenchymal stem cell, produced by the production method according to [16].
[18] A therapeutic agent for a disease selected from graft-versus-host disease (GvHD), acute respiratory distress syndrome (ARDS), asthma, serious heart failure, myocardial infarction, cerebral infarction, traumatic brain injury, brain tumor, spinal cord injury, critical limb ischemia, diabetic foot ulcer, hepatic cirrhosis, acute liver failure, chronic liver failure, Crohn's disease, sepsis, viral infection, epidermolysis bullosa, diabetes, diabetic organ dysfunction, atopic dermatitis, hypersensitivity, severe combined immunodeficiency syndrome, multiple myeloma, Kawasaki disease, scleroderma, alopecia, autoimmune hepatitis, lupus nephritis, aplastic anemia, rheumatoid arthritis, systemic lupus erythematosus, Sjogren's syndrome, psoriasis, hip osteoarthritis, knee osteoarthritis (OA), intervertebral disc degeneration, complicated skin and skin soft tissue infection, bacterial pneumonia, viral pneumonia, Pseudomonas aeruginosa infection, acquired immunodeficiency syndrome, rabies, influenza, osteonecrosis, osteopenia, alveolar bone loss, bone injury, osteogenesis imperfecta, spondylosyndesis, muscular atrophy, tendon injury, knee injury, muscle injury, musculoskeletal injury, osteoporosis, movement disorder disease, multiple sclerosis, cerebral palsy, dementia, optic neuritis, carpal tunnel syndrome, striatonigral degeneration, olivopontocerebellar atrophy, Tourette syndrome, facial hemiatrophy, periventricular leukomalacia, spinocerebellar ataxia, schizophrenia, bipolar disorder, major depressive disorder, anxiety disorder, adjustment disorder, alcoholism, congenital dysautonomia, encephalitis, epilepsy, spina bifida, amyotrophic lateral sclerosis, spinal muscular atrophy, primary lateral sclerosis, muscular dystrophy, Alzheimer's disease, Parkinson's disease, Fabry disease, Huntington's disease, mucopolysaccharidosis type I, Charcot-Marie-Tooth disease, neuropathic pain, peripheral neuropathy, trigeminal neuralgia, diabetic neuropathy, sciatica, spinal stenosis, hypoxic-ischemic encephalopathy, cerebral hemorrhage, breast cancer, metastatic pancreatic cancer, pancreatic ductal adenocarcinoma, gastrointestinal cancer, head and neck cancer, oral cancer, mesothelioma, metastatic non-small-cell lung cancer, melanoma, skin cancer, glioblastoma, solid cancer, adenocarcinoma, glioma, medulloblastoma, uveal melanoma, bronchiectasis, chronic Bronchus disease, respiratory distress syndrome, idiopathic pulmonary fibrosis, pulmonary malformation, pulmonary emphysema, pneumoconiosis, respiratory failure, acute lung injury, lung injury, preeclampsia, congestive heart failure, angina pectoris, myocarditis, congenital heart disease, dilated cardiomyopathy, multiple organ failure, coronary artery disease, ischemia, varicose ulcer, thromboangiitis obliterans, peripheral arterial occlusive disease, intermittent claudication, pulmonary stenosis, arteriovenous fistula, peripheral vascular disease, angiogenic disorder, atherosclerosis, corneal injury, keratitis, corneal dystrophy, corneal ulcer, diabetic retinopathy, glaucoma, retinitis pigmentosa, dry age-related macular degeneration, xerophthalmia, corneal edema, wet age-related macular degeneration, fistula, wound healing, obesity, Pitt-Hopkins syndrome, pressure ulcer, leg ulcer, skin ulcer, insulin-dependent diabetes mellitus, non-insulin-dependent diabetes mellitus, hearing loss, perianal fistula, rectal fistula, anal fistula, rectovaginal fistula, alcoholic liver disease, pancreatitis, biliary atresia, gastrointestinal bleeding, primary sclerosing cholangitis, proctitis, esophageal injury, liver injury, inflammatory bowel disease, ulcerative colitis, premature ovarian failure, ovarian cancer, oligospermia, testicular disease, erectile dysfunction, penile induration, uterine injury, renal failure, diabetic nephropathy, renal injury, renal fibrosis, interstitial cystitis, IgA nephropathy, stress urinary incontinence, urge urinary incontinence, C3 glomerulopathy, thalassemia, leukemia, systemic inflammatory response syndrome, radiation sickness, skin burn injury, reperfusion injury, syndrome X, metabolic disease, oral mucositis, periodontal disease, gum disease, and autism spectrum disorder; or a pharmaceutical composition for applied use selected from suppression of rejection associated with transplantation in regenerative therapy, support for transplantation (organ transplantation, hematopoietic stem cell transplantation, bone marrow transplantation, corneal transplantation, islet transplantation), improvement in aging, and use for vaccine, the therapeutic agent or pharmaceutical composition comprising the cell population comprising the mesenchymal stem cell according to claim 17 as an active ingredient.
[19] A method for determining a progress state of differentiation of a pluripotent stem cell when inducing differentiation of the pluripotent stem cell into a neural crest cell and/or a neural crest progenitor cell, the method comprising:
   (I) a step of collecting a part of a cell population during culturing at a desired time point;
   (II) a step of measuring the expression level of a predetermined gene collected from the cell population;
   (III) a step of comparing the expression level of the measured gene with a threshold; and
   (IV) a step of determining that when the expression level of the measured gene is equal to or higher than the threshold, the cultured cell population at the desired time point is capable of differentiating into the neural crest cell and/or the neural crest progenitor cell.
[20] The method according to [19], wherein the predetermined gene comprises one or more genes that has enhanced expression after inducing differentiation, and/or one or more genes that has enhanced expression during an expansion culturing period.
[21] The method according to [20], wherein the one or more genes that has enhanced expression after inducing differentiation comprise one or more selected from the group consisting of SOX10, RHOB, FOXD3, and NOTCH1, and the one or more genes that has enhanced expression during the expansion culturing period comprise one or more selected from the group consisting of SOX9, TFAP2A, NGFR, TWIST1, and EDN3.
[22] The method according to any of [19] to [21], wherein the desired time point is any time point in the step of inducing differentiation of the pluripotent stem cell into the neural crest cell and/or the neural crest progenitor cell to obtain a cell population comprising the neural crest cell and/or the neural crest progenitor cell, or
   the desired time point is any time point in the step of selectively culturing a neural crest cell in the cell population comprising the neural crest cell and/or the neural crest progenitor cell.
[23] Use of one or more extracellular matrices selected from the group consisting of laminin with its α chain being an α1 chain and its β chain being a β1 chain, laminin with its α chain being an α2 chain and its β chain being a β1 chain, laminin with its α chain being an α2 chain and its β chain being a β2 chain, and laminin with its α chain being an α5 chain and its β chain being a β1 chain, and extracellular matrices comprising integrin-binding sites of these laminins, in selective culturing of a neural crest cell in a cell population comprising a neural crest cell and/or a neural crest progenitor cell.

### Advantageous Effects of Invention

The method for culturing a neural crest cell and the method for producing a cell population containing the neural crest cell according to the present invention can provide a cell population containing a neural crest cell of high purity without sorting a neural crest cell from the cell population containing a neural crest cell after inducing differentiation by flow cytometry or the like. Further, the method can provide a cell population containing a mesenchymal stem cell of high purity, by inducing differentiation of the obtained cell population into a mesenchymal stem cell.

The method for determining a progress state of differentiation of a pluripotent stem cell into a neural crest cell and/or a neural crest progenitor cell according to the present invention, in particular based on the expression level of a predetermined gene can determine a progress state of differentiation of the cultured cell population at a desired time point into the neural crest cell and/or the neural crest progenitor cell. In addition, according to the present invention, it becomes possible to select a neural crest cell to obtain a neural crest cell of a desired cell state.

### Brief Description of Drawings

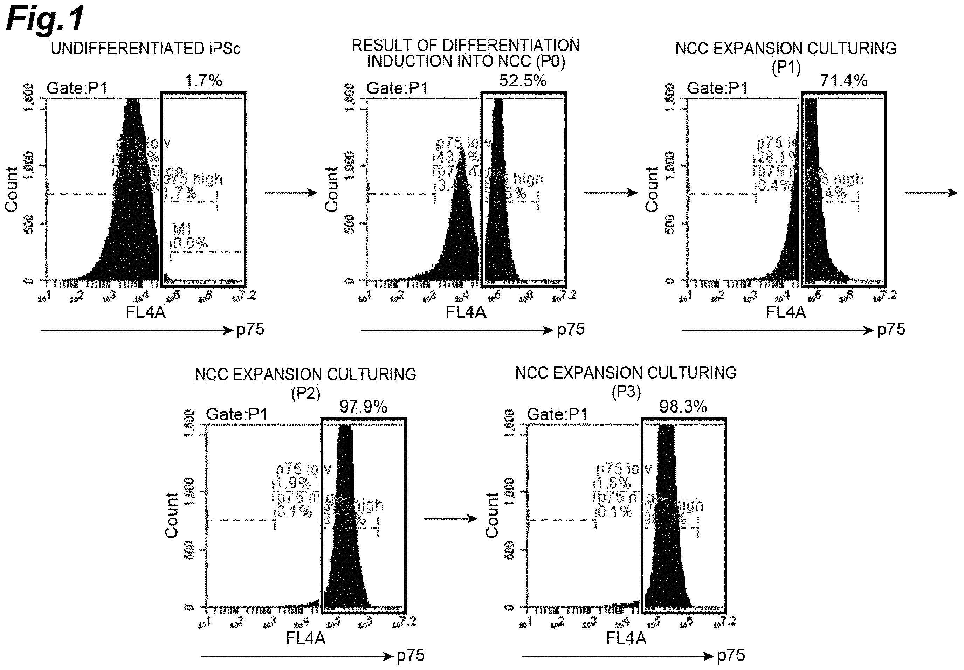

[Figure 1] Figure 1 is diagrams showing results of the expression of neural crest cell marker p75 at the time of inducing differentiation of an iPS cell into a neural crest cell, and at the time of expansion culturing of the differentiation-induced neural crest cell in Example 1, observed over time by flow cytometry.

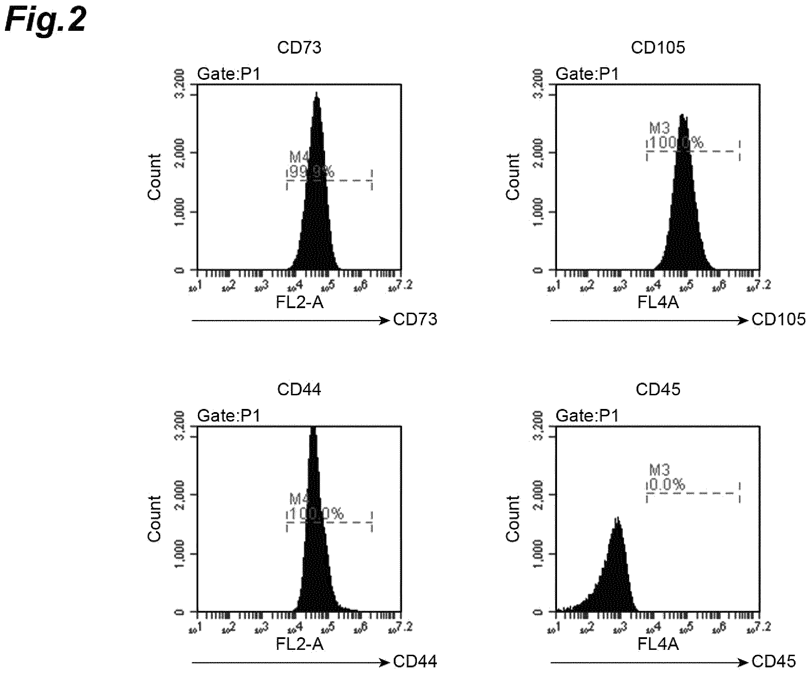

[Figure 2] Figure 2 is diagrams showing results of the expression of mesenchymal stem cell marker at the time of inducing differentiation of a neural crest cell into a mesenchymal stem cell in Example 1, observed by flow cytometry.

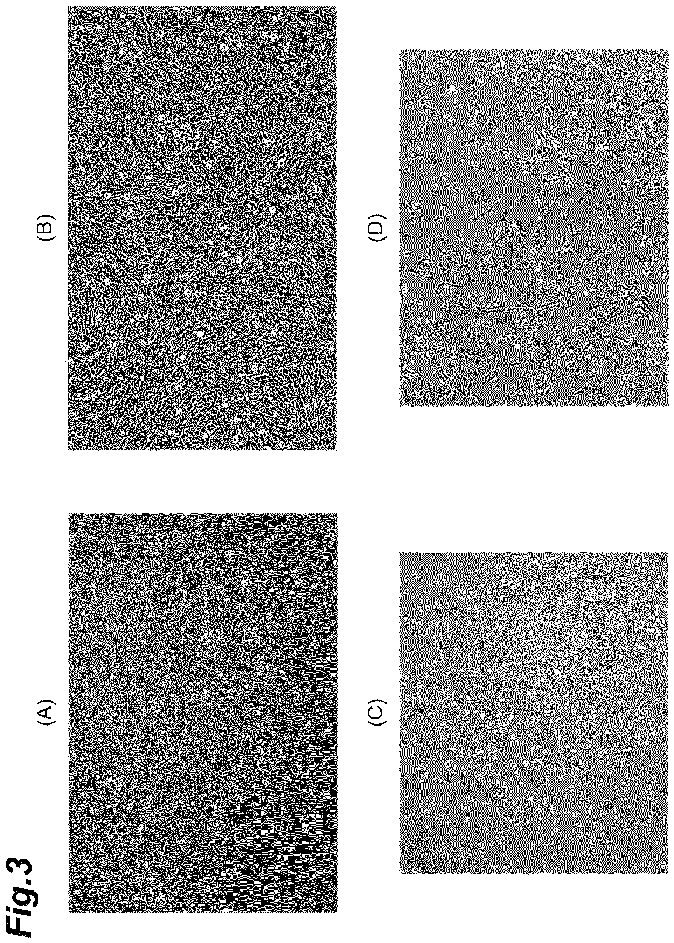

[Figure 3] Figure 3 is picture images of the neural crest cells expansion cultured on laminin 211 or laminin 221 after differentiation-induction in Example 1 and Example 2, observed by an optical microscope.

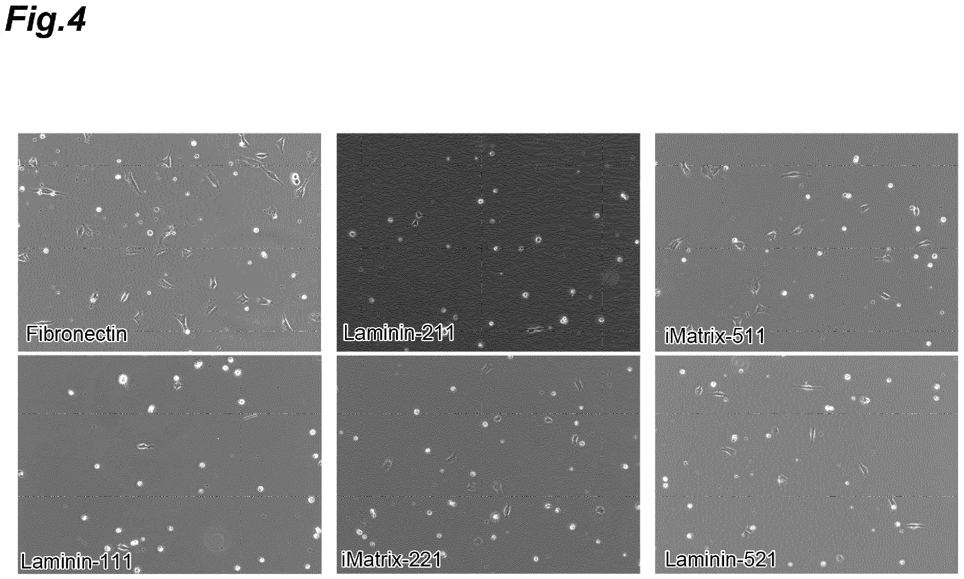

[Figure 4] Figure 4 is picture images of the states of cell adhesion of the differentiation-induced neural crest cells with respect to various extracellular matrices in Example 3, observed by an optical microscope.

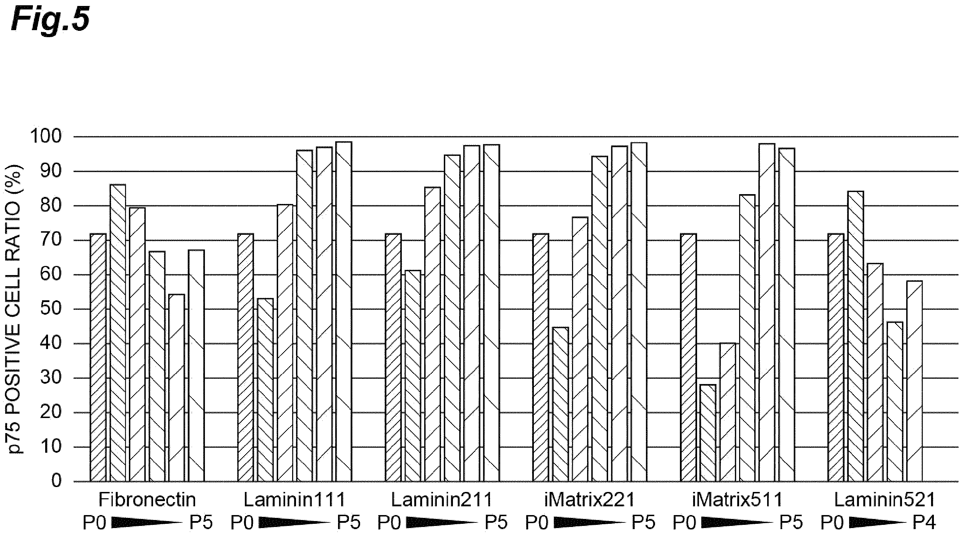

[Figure 5] Figure 5 is a graph showing results obtained by measuring, by flow cytometry, p75 positive cell ratios of the differentiation-induced neural crest cell (P0), and the cell populations of P1, P2, P3, P4, and P5 expansion cultured using various extracellular matrices in Example 3.

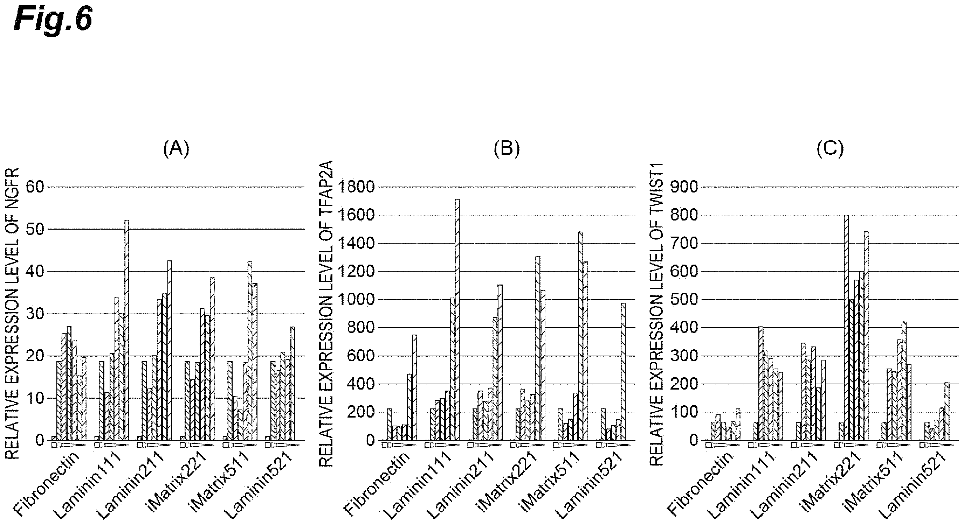

[Figure 6] Figure 6 is graphs showing results obtained by measuring, by real-time PCR, relative expression levels of NGFR (A), TFAP2A (B) and TWIST1 (C) of the differentiation-induced neural crest cell (P0), and the cell populations of P1, P2, P3, P4, and P5 expansion cultured using various extracellular matrices, relative to the iPS cells before seeding (Day 0) in Example 3.

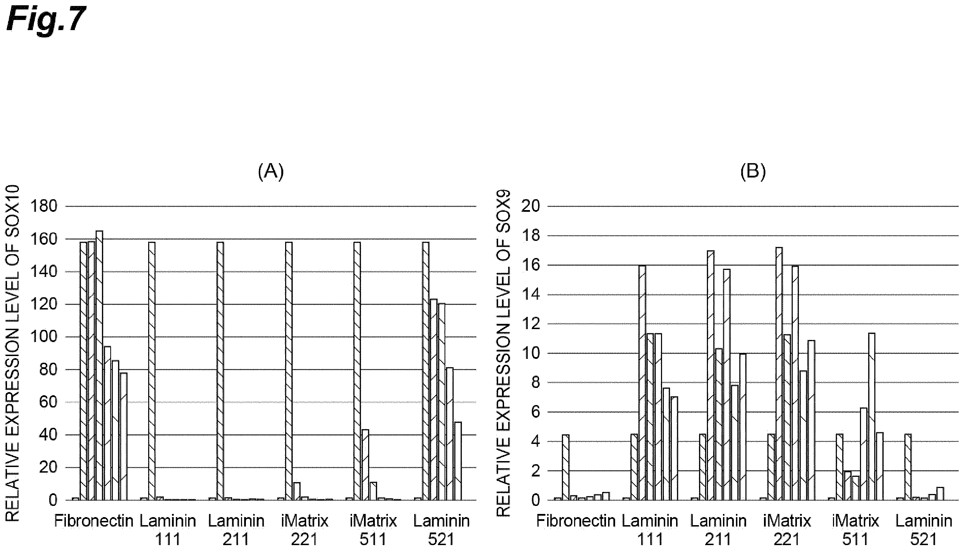

[Figure 7] Figure 7 is graphs showing results obtained by measuring, by real-time PCR, relative expression levels of SOX10 (A) and SOX9 (B) of the differentiation-induced neural crest cell (P0), and the cell populations of P1, P2, P3, P4, and P5 expansion cultured using various extracellular matrices, relative to the iPS cells before seeding (Day 0) in Example 3.

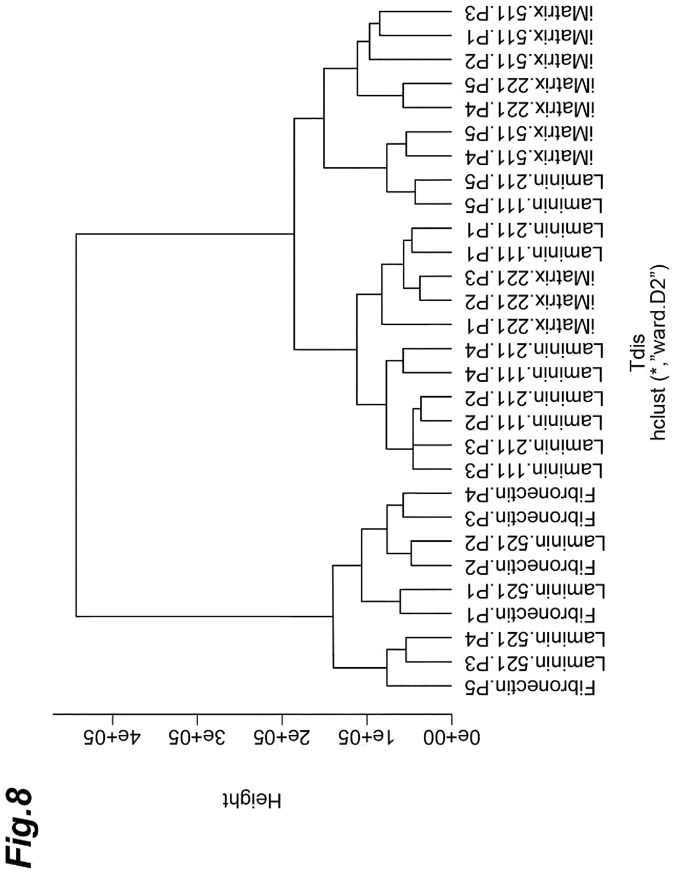

[Figure 8] Figure 8 is a dendrogram showing results obtained by conducting micro array analysis on cell populations of respective passages that are expansion cultured using various extracellular matrices, and by conducting cluster analysis based on the expression levels of the thus obtained entire genes in Example 4.

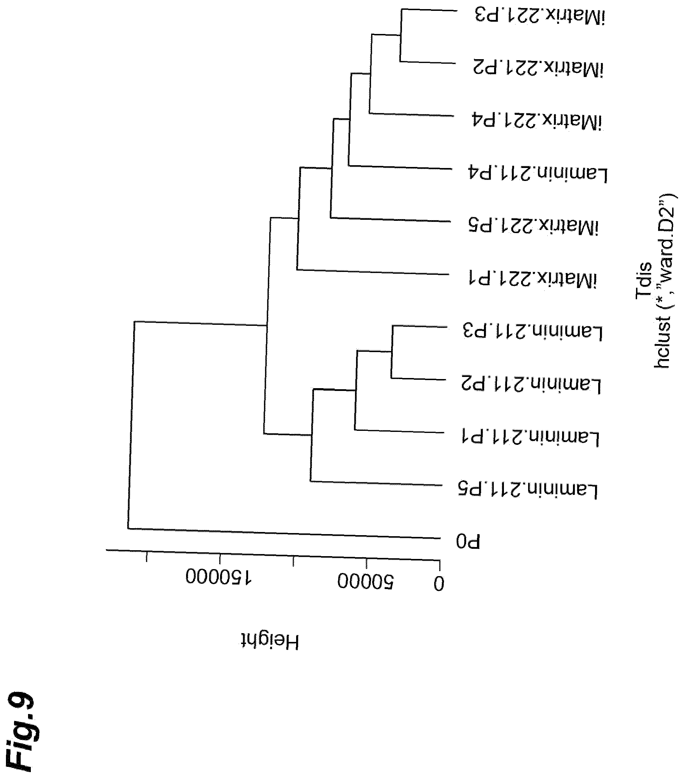

[Figure 9] Figure 9 is a dendrogram showing results obtained by conducting micro array analysis on the differentiation-induced neural crest cell (P0), and the cell populations of respective passages that are expansion cultured using two extracellular matrices, and by conducting cluster analysis based on the expression levels of the thus obtained entire genes in Example 6.

### Description of Embodiments

### [Method for Producing Cell Population containing Neural Crest Cell]

A method for producing a cell population containing neural crest cells according to an embodiment includes at least the following step (1) and step (2):
(1) a step of inducing differentiation of pluripotent stem cells into neural crest cells and/or neural crest progenitor cells to obtain a cell population containing the neural crest cells and/or the neural crest progenitor cells; and
(2) a step of adherent culturing the cell population containing the neural crest cells and/or the neural crest progenitor cells obtained in step (1) in the presence of a predetermined extracellular matrix.

Step (1) is a step of inducing differentiation of the pluripotent stem cells into the neural crest cells and/or the neural crest progenitor cells, and step (2) is a step of selectively culturing the neural crest cells in order to raise the proportion of (purify) the neural crest cells in the cell population containing the neural crest cells and/or the neural crest progenitor cells.

### <Step (1) Inducing Differentiation of Pluripotent Stem Cells into Neural Crest Cells and/or Neural Crest Progenitor Cells>

### [Pluripotent Stem Cell]

The term "stem cell" as used herein means an undifferentiated cell having differentiation potential and proliferative capacity (in particular, self-renewal capacity) with differentiation potential kept within. In the stem cells, subsets such as pluripotent stem cells, multipotent stem cells and unipotent stem cells are included according to the differentiation potential.

The pluripotent stem cell can be induced from a fertilized egg, a clone embryo, a germline stem cell, an interstitial stem cell, a somatic cell and the like. Examples of the pluripotent stem cell include embryonic stem cells (ES cells), embryonic germ cells (EG cells), and induced pluripotent stem cells (iPS cells).

The ES cell was first established in 1981, which has been applied to produce a knockout mouse since 1989. In 1998, a human ES cell was established, which is becoming available for regenerative therapy. The ES cell can be produced by culturing the inner cell mass on a feeder cell. The ES cell can also be produced by culturing in a medium containing a leukemia inhibitory factor (LIF) for a mouse and a fibroblast growth factor (bFGF) for a human, in place of the feeder cell. The methods for producing an ES cell are described, for example, in WO96/22362, WO02/101057, US5,843,780, US6,200,806, US6,280,718, and the like. The ES cell can be available from a predetermined organization, and can also be commercially purchased. For example, human ES cells, KhES-1, KhES-2 and KhES-3, are available from Institute for Frontier Medical Sciences Kyoto University. Mouse ES cells, EB5 cell line and D3 cell line, are available from Institute of Physical and Chemical Research, National Research and Development Agency and ATCC, respectively. Note that the ES cell is an ES cell established from an embryo within 14 days after fertilization.

It is possible to establish a nuclear transfer embryonic stem cell (ntES cell), one of the ES cells, from a clone embryo produced by transferring a somatic cell nucleus into an egg without a nucleus.

It is possible to produce the EG cell by culturing a primordial germ cell in a medium containing mSCF, LIF and bFGF (Cell, 70: 841-847, 1992).

The term "induced pluripotent stem cell (iPS cell)" as used herein is a cell into which pluripotency is induced by reprogramming a somatic cell by a known method or the like. Specifically, examples thereof include a cell obtained by reprogramming a fibroblast, or a differentiated somatic cell such as a peripheral blood mononuclear cell by the expression of any of combinations of a plurality of genes selected from the group of reprogrammed genes including Oct3/4, Sox2, Klf4, Myc (c-Myc, N-Myc, and L-Myc), Glis1, Nanog, Sall4, Lin28, Esrrb or the like to induce multi-differentiation capacity. Preferable examples of the combination of initialized factors includes: (1) Oct3/4, Sox2, Klf4, and Myc (e.g., c-Myc or L-Myc); and (2) Oct3/4, Sox2, Klf4, Lin28 and L-Myc (Stem Cells, 2013; 31:458-466).

The iPS cell was established by using a mouse cell by Yamanaka et al., in 2006 (Cell, 2006, 126(4), pp.663-676). The iPS cell was also established by using a human fibroblast in 2007, and has a pluripotency and self-renewal capacity in the same manner as the ES cell (Cell, 2007, 131(5), pp.861-872; Science, 2007, 318(5858), pp.1917-1920; and Nat. Biotechnol., 2008, 26(1), pp.101-106).

It is also possible to produce the iPS cell by a method for inducing an iPS cell from a somatic cell by an addition of a compound or the like, as well as the method for producing it by directly reprogramming through gene expression (Science, 2013, 341, pp.651-654).

It is also possible to obtain an established iPS cell, and for example, human iPS cell lines such as 201B7 cell, 201B7-Ff cell, 253G1 cell, 253G4 cell, 1201C1 cell, 1205D1 cell, 1210B2 cell, and 1231A3 cell which were established at Kyoto University are available from Kyoto University. As the established iPS cells for clinical use, for example, Ff-I01, Ff-I14, QHJI01 and QHJI 14 which were established at Kyoto University are available from Kyoto University. In addition, it is possible to produce the iPS cell by using a somatic cell, for example.

Examples of the somatic cell to be used in producing the iPS cell include, but not limited to, a tissue-derived fibroblast, a hematopoietic cell (e.g., peripheral blood mononuclear cell (PBMC), T-cell), a hepatic cell, a pancreatic cell, an intestinal epithelial cell, a smooth muscle cell, a dental pulp cell. Examples of the origin of the somatic cell to be used in producing the iPS cell include peripheral blood and cord blood collected from a human blood vessel, skin tissue, and a tooth.

When reprogramming through several kinds of gene expression in producing an iPS cell, a means for expressing the gene is not particularly limited. Examples thereof include an infection technique by using a viral vector (e.g., Retroviral vector, Lentiviral vector, Sendai virus vector, Adenoviral vector, or Adeno-associated viral vector), a gene transfer technique (e.g., calcium phosphate method, lipofection method, RetroNectin method, or electroporation method) by using a plasmid vector (e.g., plasmid vector or episomal vector), a gene transfer technique (e.g., calcium phosphate method, lipofection method, or electroporation method) by using an RNA vector, a protein direct infusion method (e.g., a method by using a needle, lipofection method, or electroporation method). It is also possible to use a commercially available iPSC reprogramming kit (e.g., Cyto Tune (trademark)-iPS, iPSC reprogramming kit using Sendai virus vector).

It is possible to produce the iPS cell in the presence of a feeder cell or in the absence of a feeder cell (feeder-free). When producing the iPS cell in the presence of the feeder cell, it is possible to produce the iPS cell in the presence of a factor for maintaining undifferentiation by a known method. A medium used in producing the iPS cell in a feeder-free condition is not particularly limited, and it is possible to use a medium for maintaining a known ES cell and/or iPS cell, or a medium for establishing the iPS cell in a feeder-free condition. Examples of the medium for establishing the iPS cell in the feeder-free condition include a medium for feeder-free culturing such as an Essential 8 medium (E8 medium), an Essential 6 medium, a TeSR medium, a mTeSR medium, a mTeSR-E8 medium, a Stabilized Essential 8 medium, a StemFit medium, all of which are commercially available. When producing iPS cells, using Sendai virus vector to somatic cells in a feeder-free condition leads to gene transfer of 4 factors of Oct3/4, Sox2, Klf4, and Myc (e.g., c-Myc or L-Myc), whereby it is possible to produce iPS cells.

The pluripotent stem cell in the present invention is preferably a pluripotent stem cell of a mammal, more preferably a pluripotent stem cell of a rodent (e.g., mouse or rat) or a primate (e.g., human or monkey), even more preferably a pluripotent stem cell of a primate, and particularly preferably a human iPS cell or a human ES cell.

### [Undifferentiation Maintaining Medium for Pluripotent Stem Cell]

The medium to be used for culturing pluripotent stem cells in the present invention, i.e., a medium capable of maintaining the pluripotency of the pluripotent stem cell, can be obtained by preparing a medium that is usually used for culturing animal cells as a basal medium. Examples of the basal medium for culturing pluripotent stem cells include BME medium, BGJb medium, CMRL, 1066 medium, Glasgow's Minimal Essential Medium (GMEM) medium, Improved MEM Zinc Option medium, IMDM medium, Medium 199 medium, Eagle MEM medium, αMEM medium, DMEM medium, F-12 medium, DMEM/F12 medium, IMDM/F12 medium, Ham's medium, RPMI 1640 medium, Fischer's medium, Neurobasal medium, and a mixed medium thereof.

Herein, it is desirable for the medium capable of maintaining the pluripotency of the pluripotent stem cell to be a medium containing a factor for maintaining undifferentiation (undifferentiation maintaining medium) so as to maintain an undifferentiated state of pluripotent stem cells. A commercially available medium can be used as the medium, and for example, it can be prepared by adding a undifferentiation maintaining factor, a serum replacement and, as appropriate, an nutritional source and the like to the basal medium. Specifically, it can be prepared by adding bFGF, KSR, non-essential amino acid (NEAA), L-glutamine and 2-mercaptoethanol to a DMEM/F12 medium. Further, it is also possible to use a feeder-free medium such as the above-mentioned Essential 8 medium (E8 medium), Essential 6 medium, TeSR medium, mTeSR medium, mTeSR-E8 medium, Stabilized Essential 8 medium, StemFit medium, or the like.

In view of ensuring safety and ensuring a suitable quality by minimizing the difference between lots, for example, it is preferable for the medium used for culturing pluripotent stem cells to be a xeno-free medium, and more preferable to be a medium which contains no animal-derived ingredient, and which composition is chemically-known. Accordingly, it is desirable for the medium used for culturing pluripotent stem cells to be a feeder-free medium or a serum-free medium.

### [Method for Inducing Differentiation into Neural Crest Cells]

The neural crest is a structure transiently formed between the surface ectoderm and the neural plate during early vertebrate development, and is also referred to as the "fourth germ layer" because it differentiates into various cell strains. The neural crest cells (NCC) are a cell group that delaminates from neural crest cells, and widely migrates in the developing embryo after the epithelial mesenchymal transition. By spreading over a specific region in the embryo, the neural crest cells differentiate into region-characteristic various cell strains such as a peripheral nerve, cartilage, a smooth muscle, or the like. Since the neural crest cells are a cell population that transiently develops, it was difficult to collect from the developing embryo; however, there was lately reported development of a method for inducing differentiation of pluripotent stem cells of a mouse or human into neural crest cells, whereby it has become possible to obtain. The neural crest progenitor cell is a generic name of cell groups destined to differentiate into neural crest cell.

The terms "neural crest cell" and "neural crest progenitor cell" as used herein refer to a neural crest cell and neural crest progenitor cell that are obtained from an embryo during its development, and a neural crest cell and neural crest progenitor cell obtained by an induction-differentiation method, and it is possible to identify the neural crest cell and the neural crest progenitor cell by a marker that expresses in the neural crest cells and the neural crest progenitor cells. Examples of the neural crest cell marker include TFAP2A, NGFR (synonym for p75), TWIST (TWIST1 or TWIST2), and EDN3, and examples of the neural crest progenitor cell marker include FOXD3, PAX3, and ZIC1.

Herein, the neural crest progenitor cell may be included in the "cell population containing the neural crest cells". In addition, the "cell population containing the neural crest cells" has a possibility to contain neural crest stem cells.

The neural crest cells and/or the neural crest progenitor cells can be obtained by inducing differentiation of pluripotent stem cells. The induction-differentiation method is not particularly limited, but for example, the methods described in Patent Literatures 1 to 4, and Non Patent Literatures 1 and 2 can be used. Further, considering the efficiency of induction-differentiation, it is preferable to culture a cell population containing pluripotent stem cells in a medium containing at least one SMAD signaling inhibitor and at least one Wnt signaling activator.

The SMAD signaling inhibitor is, for example, an inhibitor of TGFβ superfamily that can inhibit SMAD signaling by inhibiting phosphorylation of SMAD upstream of the SMAD signal, and specific examples thereof include a TGFβ inhibitor and a BMP inhibitor. The SMAD signaling inhibitor can be obtained by using at least one TGFβ inhibitor and at least one BMP inhibitor in combination appropriately. It is preferable for the SMAD signaling inhibitor to include at least one TGFβ inhibitor.

Herein, the TGFβ inhibitor is not particularly limited as long as it can suppress the signaling that is mediated by TGFβ, and it can be any of a nucleic acid, protein, and a low molecular weight organic compound. Examples of the TGFβ inhibitor include a substance for directly acting on TGFβ (e.g., protein, antibiotic, aptamer, or the like), a substance for suppressing the expression of a gene that encodes TGFβ (e.g., antisense oligonucleotide, siRNA, or the like), a substance for inhibiting the bonding between a TGFβ receptor and TGFβ, a substance for inhibiting the bioactivity due to TGFβ receptor signaling (e.g., inhibitor of TGFβ receptor, or the like). Examples of the TGFβ inhibitor include a substance for inhibiting the bonding to ALK family, which is a receptor, or a substance for inhibiting the phosphorylation of SMAD by the ALK family.

Herein, specific examples of the TGFβ inhibitor include SB431542, SB202190 (R. K. Lindemann et al., Mol. Cancer 2:20 (2003)), SB505124 (GlaxoSmithKline), NPC30345, SD093, SD908, SD208 (Scios), LY2109761, LY364947, LY580276 (Lilly Research Laboratories), A-83-01 (WO2009146408), Galunisertib (LY2157299), LY3200882, SB525334, GW788388, RepSox, Lefty-1 (examples of the NCBI Accession No. include mouse: NM 010094 and human: NM_020997), Lefty-2 (examples of the NCBI Accession No. include mouse: NM_177099, human: NM_003240 and NM_001172425), and derivatives thereof, and it is possible to use at least one TGFβ inhibitor. It is preferable for the TGFβ inhibitor to be one or more selected from the group consisting of SB431542 and A83-01 that are known as an inhibitor of TGFβ receptor (ALK5) and an activin receptor (ALK4/7).

The concentration of the TGFβ inhibitor in the medium can be appropriately set within a range capable of suppressing the signaling that is mediated by TGFβ. For example, the concentration of SB431542 is not particularly limited as long as it is the concentration capable of inhibiting ALK5, and for example, it is 100 nM to 100 µM, preferably 500 nM to 30 µM, and further preferably 1 µM to 20 µM. In a case of other TGFP inhibitors, the concentration exhibiting the ALK inhibitory activity or TGFβ inhibitory activity that correspond to SB431542 at the aforementioned concentration.

The BMP inhibitor is not particularly limited as long as it is an inhibitor involved in the inhibition of BMP signaling that mediates the bonding between bone morphogenetic protein (BMP) and a BMP receptor (type I or type II), and it can be any of a nucleic acid, protein, and a low molecular weight organic compound. Examples of the BMP inhibitor include a substance for directly acting on BMP (e.g., protein, antibody, aptamer, or the like), a substance for suppressing the expression of a gene that encodes BMP (e.g., antisense oligonucleotide, siRNA, or the like), a substance having the biological activity that inhibits the above BMP signaling by inhibiting the bonding of BMP to a BMP receptor, a substance for inhibiting the bioactivity due to BMP receptor signaling (e.g., inhibitor of BMP receptor, or the like), and BMP type-I receptor kinase inhibitor.

Specific examples of that having a biological activity of inhibiting the above BMP signaling by inhibiting the bonding of BMP to the BMP receptor include Dorsomorphin, Noggin and derivatives thereof. Further, specific examples of the BMP type-I receptor kinase inhibitor include LDN-193189 (i.e., 4-(6-(4-(piperazin-1-yl)phenyl)pyrazolo[1,5-a]pyrimidin-3-yl)quinoline and derivatives thereof (e.g., LDN-212854). Examples of other BMP inhibitors include K02288, Chordin and Follistatin.

It is preferable for the BMP inhibitor to be one or more selected from the group consisting of Noggin, LDN-193189, Dorsomorphin and K02288 (PLoS One. 2013; 8(4): e62721.).

The concentration of the BMP inhibitor in the medium can be appropriately set within a range capable of suppressing the signaling that is mediated by BMP, for example. For example, the concentration of LDN-193189 is not particularly limited as long as it is the concentration capable of inhibiting BMP type-I receptor kinase, and for example, it is 0.1 nM to 10 µM or 0.5 nM to 10 µM, preferably 10 nM to 5 µM, further preferably 50 nM to 3 µM, and further more preferably 50 nM to 1 µM. In a case of other BMP inhibitors, the concentration exhibiting the BMP type-I receptor kinase inhibitory activity or BMP inhibitory activity that correspond to LDN-193189 at the aforementioned concentration can be appropriately set.

The Wnt signaling activator is not particularly limited as long as it can activate the signaling that is mediated by Wnt. Examples of the Wnt signaling activator include Wnt protein (Wnt3a or the like), Wnt agonist, Dkk (inhibitor of Wnt signaling inhibitory protein), GSK3β (glycogen synthase kinase 3β) inhibitor, and R-Spondin.

Herein, the GSK3β inhibitor is defined as a substance for inhibiting kinase activity of glycogen synthase kinase (GSK) 3β protein, and specific examples thereof include indirubin derivatives such as BIO (alias: GSK-3β inhibitor IX; 6-bromoindirubin-3'-oxime), maleimide derivatives such as SB216763, α-bromo methyl ketone compounds such as GSK-3β inhibitor VII, cell-penetrating phosphorylated peptide such as CHIR99021, kenpaullone, L803-mts, and derivatives thereof.

Herein, it is preferable for the Wnt signaling activator to be a GSK3β inhibitor, and to be one or more selected from the group consisting of CHIR99021, BIO, kenpaullone, SB216763 and L803-mts that are GSK3β inhibitors.

The concentration of the Wnt signaling activator in the medium can be appropriately set within a range capable of facilitating the signaling that is mediated by Wnt protein (Wnt3a, or the like). for example. For example, the concentration of CHIR99021, is not particularly limited as long as it is the concentration capable of inhibiting GSK3β, and for example, it is 100 nM to 100 µM, preferably 500 nM to 30 µM, and further preferably 1 µM to 10 µM. In a case of other Wnt signaling activators, the concentration exhibiting facilitation of the signaling that is mediated by the GSK3β inhibitory activity or Wnt protein (Wnt3a or the like) that correspond to CHIR99021 at the aforementioned concentration.

In step (1), the pluripotent stem cells are cultured in the absence of a feeder cell. The absence of a feeder cell is interchangeable with feeder-free, and indicates a state where the feeder cell does not exist in the medium. Examples of the culture condition in the absence of a feeder cell include culture conditions without addition of a feeder cell such as fibroblast, SNL cell or STO cell.

The term "neural crest cell differentiation inducing medium" as used herein is a medium that contains a differentiation inducing factor from pluripotent stem cells into neural crest cells, and that can induce differentiation into neural crest cells and/or neural crest progenitor cell by culturing pluripotent stem cells in this medium. The neural crest cell differentiation inducing medium may be a feeder-free medium suitable for inducing differentiation into neural crest cells and for feeder- free culturing pluripotent stem cells. The neural crest cell differentiation inducing medium can be suitably prepared by, for example, adding an adding factor such as a serum replacement and a nutritional source and the like to the basal medium as appropriate, and further adding a differentiation inducing factor thereto.

Step (1) of the present invention is inducing differentiation into neural crest cells by culturing pluripotent stem cells in the neural crest cell differentiation inducing medium so as to be able to obtain a cell population containing neural crest cells and/or neural crest progenitor cells. In other words, step (1) is not limited to be comprised of one step as long as the neural crest cells and/or the neural crest progenitor cells can be obtained in step (1) as a whole, and step (1) can be comprised of a plurality of subdivided substeps. For example, step (1) can be comprised of 2 to 5 substeps, and preferably 2 to 3 substeps. More specifically, step (1) can be comprised of (i) a plurality of substeps in which all of the neural crest cell differentiation inducing medium to be used in each substep has the same composition, but the respective culturing conditions such as the culturing temperature and the oxygen partial pressure are different; (ii) a plurality of substeps in which the types and/or concentrations of one or more differentiation inducing factors are different; and (iii) a combination of the above (i) and (ii).

In other words, the timing of processing pluripotent stem cells with the differentiation inducing factor necessary for inducing differentiation into neural crest cells may be appropriately set, and when using a plurality of differentiation inducing factors, the timing may be the same or different. In addition, the concentration of the differentiation inducing factor may be appropriately set, and it may be constant, or may gradually increase or decrease.

The basal medium to be used in step (1) is not particularly limited as long as it can culture cells, and it is possible to use a basal medium that is commercially available as a basal medium for cell growth, as appropriate. Examples of the basal medium for cell growth include media that can be used for animal cell culture such as BME medium, BGJb medium, CMRL, 1066 medium, Glasgow's MEM (GMEM) medium, Improved MEM Zinc Option medium, IMDM medium, Medium 199 medium, Eagle MEM medium, αMEM medium, DMEM medium, F-12 medium, DMEM/F-12 medium, IMDM/F12 medium, Ham's medium, RPMI 1640 medium, Fischer's medium, CDM medium, or a mixed medium thereof. Here, the CDM medium means a medium consisting of the ingredients identified as chemical substances.

In these basal media, included are ingredients necessary for cells to survive and/or proliferate, such as carbon source, e.g., glucide and amino acids, vitamins, inorganic salts, and/or serums or serum replacements. In addition, it is preferable to be a chemically defined medium without of serum replacement or without serum.

For example, the CDM medium is preferably used as the basal medium used in step (1). Specific examples of the CDM medium include a medium including IMDM/Ham's F-12 1:1 (GIBCO, USA) and 1× Chemically Defined Lipid Concentrate (GIBCO).

As the neural crest cell differentiation inducing medium, for example, it is also possible to use a medium in which a part or whole of the factors necessary for undifferentiation maintenance (undifferentiation maintaining factors) are removed from a medium for maintenance culturing pluripotent stem cells while remaining in the undifferentiated state (undifferentiation maintaining medium), or a medium in which the concentrations of a part or whole of the undifferentiation maintaining factors are lowered to an effective concentration or less, as described later. Such a medium can be prepared by formulating so as to include the ingredients similar to those in the medium, for example. Alternatively, it is possible to use a medium obtained by adding a serum replacement or a nutritional factor such as hormones to a basal medium such as DMEM, for example. Alternatively, there is a case where a undifferentiation maintaining medium is marketed as a kit for use by appropriately mixing a plurality of liquid solutions, and in this case, it is possible to prepare a neural crest cell differentiation inducing medium by formulating without adding liquid solution containing a undifferentiation maintaining factor. For example, in a case of StemFit AK03N (Ajinomoto Healthy Supply Co., Inc.), in which Liquid A, Liquid B, and Liquid C are separately prepared, it is therefore possible to prepare a neural crest cell differentiation inducing medium using only Liquid A and Liquid B, but not using Liquid C that contains FGF2 (also referred to bFGF).

As an aspect, differentiation inducing factors such as the SMAD signaling inhibitor and the Wnt signaling activator are contained in the neural crest cell differentiation inducing medium, and it is preferable that ingredients inhibiting or antagonizing the effects of the differentiation inducing factors include to the extent of not affecting step (1), for example, at a concentration within a range without affecting thereon, or such ingredients do not include.

Examples of the neural crest cell differentiation inducing medium used in step (1) include a medium in which induction from the neural crest cells to neural cells is suppressed. Examples of the neural crest cell differentiation inducing medium used in step (1) include a basal medium (e.g., CDM medium) containing the SMAD signaling inhibitor and/or the Wnt signaling activator.

Specific examples of the neural crest cell differentiation inducing medium used in step (1) include a medium obtained by adding, to a CDM medium (IMDM/Ham's F-12 1:1 (GIBCO, USA), 1 × Chemically Defined Lipid Concentrate (GIBCO) including Optiferrin (InVitria, Inc., USA) (may be 0.1 to 1000 µg/ml, or 1 to 100 µg/ml, and preferably 10 to 30 µg/ml), insulin (Sigma-Aldrich Corp., USA) (may be 0.1 to 1000 µg/ml, or 1 to 100 µg/ml, and preferably 2 to 20 µg/ml), monothioglycerol (maybe 0.01 to 10 mM, or 0.1 to 1 mM, and preferably 0.3 to 0.6 mM) (FUJIFILM Wako Pure Chemical Corporation)), KSR (GIBCO) (may be 1 to 60% by volume, and preferably 10 to 30% by volume), SB431542 (FUJIFILM Wako Pure Chemical Corporation) (may be 0.1 to 1000 µM, or 1 to 500 µM, and preferably 2 to 20 µM), and CHIR99021 (FUJIFILM Wako Pure Chemical Corporation) (may be 0.01 to 100 µM, or 0.01 to 50 µM, and preferably 0.1 to 3 µM). Note that, in the production method of the present invention, the CDM medium is a medium in which induction from neural crest cells to neural cells is suppressed, and therefore, it is a medium suitable for maintaining the neural crest cells.

In step (1), it is preferable that a culture surface of a culture container, in other words, a surface on which a cell population adheres be coated with an extracellular matrix suitable for inducing differentiation into neural crest cells. Such an extracellular matrix is not particularly limited as long as it is easy for the differentiation-induced neural crest cells to be adhered thereon, but examples thereof include laminin and fibronectin. Among these, laminin 511 and fibronectin are preferably used. For example, in the case of laminin 511, the amount to be coated may be 0.001 to 30 µg/cm², and it is preferable to be 0.01 to 10 µg/cm², or 0.1 to 1 µg/cm², based on the culture surface area.

Examples of the culture container include a culture container that is usually commercially available, for example, a 6-well plate (bottom surface area of about 9.5 cm², and inner diameter of about 35 mm), a 12-well plate (bottom surface area of about 3.8 cm², and inner diameter of about 23 mm), a 24-well plate (bottom surface area of about 1.88 cm², and inner diameter of about 16 mm), a 48-well plate (bottom surface area of about 1.0 cm², and inner diameter of about 11 mm), a 96-well plate (bottom surface area of about 0.35 cm², and inner diameter of about 6 to 8 mm), a 384-well plate (bottom surface area of about 0.1 cm², and inner diameter of about 3 to 4 mm), which are flat-bottomed. Preferable is a flat-bottomed 6-well plate.

When culturing using a 6-well plate, a cell suspension solution having a uniform cell density is dispensed at an equal amount to each hole of the culture container. The cell density may be 0.1 × 10⁵ cells/well to 5.0 × 10⁵ cells/well, and preferably it is 0.2 × 10⁵ cells/well to 2.0 × 10⁵ cells/well.

Culturing in step (1) may be conducted for about 6 to 20 days, preferably about 7 to 18 days, and further preferably 10 to 18 days. The culturing duration can be decided depending on a desired cell differentiation level. The cell differentiation level that shows how much the differentiation-induced cells were differentiated can be decided from the measurement by a differentiation marker, and for example, it can be performed by increasing the expression level of p75 using FACS, or by increasing the expression level of at least one of TFAP2A, NGFR, EDN3 and TWIST1 using PCR. The increased expression level indicates a case where the expression level increases within the limits of error or more after a certain period of time has elapsed from a specific time point.

The culturing conditions of the culture in step (1) is not particularly limited as long as the conditions are, for example, a temperature of 35 to 37°C, preferably 37°C, a humidity of 90 to 95%, preferably 95%, and a CO² concentration of 3 to 5%, preferably 5%, and further, it is preferable to be under hypoxia culturing conditions. The term "hypoxia culturing conditions" as used herein means conditions for culturing cells at an oxygen concentration lower than the oxygen concentration in the air (about 21%). Here, the oxygen concentration means a concentration of oxygen in the air in which the medium containing cells comes into contact in a culture apparatus. The oxygen concentration is not particularly limited as long as it is 20% or less, and preferably within a range from 3 to 10%, more preferably within a range from 4 to 6%, and most preferably 5%.

Note that, before the differentiation, that is, before step (1), maintenance culturing of pluripotent stem cells in a undifferentiation maintaining medium may be performed. The undifferentiation maintaining medium is a medium that contains factors necessary for maintaining an undifferentiated state (undifferentiation maintaining factor), and that can maintain the pluripotency. Examples of the undifferentiation maintaining factors include fibroblast growth factors, transforming growth factor beta (TGFβ family) factors, and activin A. The term "fibroblast growth factors (FGF)" as used herein means a factor for activating FGF signals by acting on a fibroblast growth factor receptor. Examples of the FGF include FGF2 (also referred to as bFGF), FGF4 and FGF8. Note that the undifferentiation maintaining medium is as defined above.

As an aspect, the cell population containing neural crest cells and/or neural crest progenitor cells obtained in step (1) expresses at least one or more of the neural crest cell markers and the neural crest progenitor cell markers higher than iPS cells.

As an aspect, the cell population containing neural crest cells and/or neural crest progenitor cells obtained in step (1) has a cell proportion in which at least one or more of the neural crest cell markers and the neural crest progenitor cell markers are positive, of about 1% or more, preferably 10% or more, further preferably 30% or more, and more preferably 40% or more, based on the total number of cells.

As an aspect, the cell population containing neural crest cells and/or neural crest progenitor cells obtained in step (1) is a cell population containing neural crest cells. In this case, the proportion of p75 positive cells (i.e., neural crest cells) is about 1% or more, preferably 10% or more, further preferably 30% or more, and more preferably 40% or more, based on the total number of cells.

### <Step (2) Selectively Culturing Neural Crest Cell>

A method for selectively culturing a neural crest cell of an embodiment, that is, the above step (2), includes a culturing step of adherent culturing a cell population containing the neural crest cell and/or the neural crest progenitor cell, in the presence of one or more extracellular matrices selected from the group consisting of a laminin with its α chain being an α1 chain and its β chain being a β1 chain, a laminin with its α chain being an α2 chain and its β chain being a β1 chain, a laminin with its α chain being an α2 chain and its β chain being a β2 chain, and a laminin with its α chain being an α5 chain and its β chain being a β1 chain, and extracellular matrices containing integrin-binding sites of these laminins. Herein, the step (2) may be referred to as expansion culturing.

The culturing target in step (2) is the cell population containing neural crest cells and/or neural crest progenitor cells obtained in step (1), and, in this case, is subjected to step (2) as it is without being sorted or selected with a neural crest cell marker on the cell population obtained in step (1). It is because step (2) can selectively culture neural crest cells for the proliferation. As shown in Examples to be described later, it is possible to obtain neural crest cells in a cell state with a certain tendency by adherent culturing the cell population containing neural crest cells and/or neural crest progenitor cells in the presence of the aforementioned predetermined extracellular matrices.

The term "cell state with a certain tendency" as used herein means that gene expression states have a certain tendency. Since the gene expression states have a certain tendency, it is possible to perform microarray, RNA sequencing or the like on a plurality of independent cell population samples containing neural crest cells and/or neural crest progenitor cells obtained by the same culturing method, and to analyze gene profiling similarities by a cluster analysis or the like.

The laminin as used herein is a protein constituting the basement membrane in extracellular matrix, and has a heterotrimer structure having each one of the α chain, β chain, and γ chain. The α chain includes 5 kinds of α1 chain, α2 chain, α3 chain, α4 chain and α5 chain, the β chain includes 3 kinds of β1 chain, β2 chain and β3 chain, and the γ chain includes 3 kinds of γ1 chain, γ2 chain and γ3 chain. Various laminins are formed by the combinations thereof. For example, laminin 511 is composed of α5 chain, β1 chain and γ1 chain. Laminin is a cell adhesion molecule and binds to a cell surface receptor. There is integrin as a laminin receptor. There is an integrin-binding site in laminin, and a fragment of laminin that has the integrin-binding site is known as an E8 fragment.

The extracellular matrix in step (2) may be one or more laminins selected from the group consisting of a laminin with its α chain being an α1 chain and its β chain being a β1 chain (i.e., laminin 111 and laminin 112), a laminin with its α chain being an α2 chain and its β chain being a β1 chain (i.e., laminin 211 and laminin 212), a laminin with its α chain being an α2 chain and its β chain being a β2 chain (i.e., laminin 221 and laminin 222), and a laminin with its α chain being an α5 chain and its β chain being a β1 chain (i.e., laminin 511 and laminin 512), and further, it is preferable for the γ chain of the laminins to be γ1 chain, for example, it may be laminin 221, laminin 211, laminin 111, and laminin 511, and in particular, it is preferable to be laminin 221.

The extracellular matrix in step (2) may also be an extracellular matrix including the integrin-binding site of the above laminin, and it is preferable for the integrin-binding site of the laminin to be an E8 fragment including an integrin-binding site and in particular, it is preferable to be an E8 fragment of laminin 221. The extracellular matrix including the integrin-binding site of laminin may be a laminin fragment including an integrin-binding site (e.g., E8 fragment), and other amino acid residues or peptides that cannot affect the integrin-binding capacity or that can enhance the integrin-binding capacity may be peptides or proteins obtained by binding to the integrin-binding site of laminin (e.g., E8 fragment).

By using the aforementioned predetermined extracellular matrix in step (2), it is possible to selectively engraft and proliferate the neural crest cells in the cell population containing neural crest cells and/or neural crest progenitor cells, and further, to selectively engraft the neural crest progenitor cells that are likely to be differentiation-induced into neural crest cells to facilitate proliferation and/or induction-differentiation. As a result, the proportion of neural crest cells with respect to the total number of cells in the cell population increases, and thereby it is possible to enrich and purify the neural crest cells.

The adherent culture in step (2) is to adherent culture the cell population containing neural crest cells and/or neural crest progenitor cells in the presence of the aforementioned predetermined extracellular matrix. Being in the presence of the predetermined extracellular matrix may be present in a state in which the culture surface with the cell population adhered thereon is coated with the extracellular matrix, or may be present in a state in which the predetermined extracellular matrix is added to the medium, and further, may be present in both states. Among these, preferable is to be present in a state in which the culture surface with the cell population adhered thereon is coated with the extracellular matrix. In a case of coating, at least a part of the culture surface (e.g., 50% or more, 60% or more, 70% or more, 80% or more, 90% or more, or 95% or more) may be coated with the extracellular matrix, and the amount to be coated in cases of laminin 221, laminin 211, laminin 111, laminin 511 and the E8 fragments thereof, for example, may be 0.001 to 30 µg/cm² and it is preferable to be 0.01 to 10 µg/cm², or 0.1 to 2 µg/cm², based on the culture surface area. In a case of adding to the medium, the amount to be coated in cases of laminin 221, laminin 211, laminin 111, laminin 511 and the E8 fragments thereof, for example, may be 0.1 to 1000 ng/cm² and it is preferable to be 1 to 100 ng/cm², or 2 to 20 ng/cm², based on the culture surface area.

The medium used in step (2) is not particularly limited as long as it is a medium suitable for maintaining neural crest cells so as not to differentiate the neural crest cells, but it is preferable to be a medium suitable for maintaining and proliferating the neural crest cells. The "medium suitable for maintaining neural crest cells" may be any medium obtained by adding, to the basal medium in step (1), ingredients necessary for maintaining neural crest cells without causing cell death or further differentiating. In addition, in order to reduce the impact on cells, it is preferable for the basal medium in step (2) to be the same as the basal medium used in step (1). For example, a serum replacement and, as appropriate, a nutritional source and the like can be added to the basal medium in step (1), and examples of such ingredients include albumin, BSA, HSA, FBS, KSR, TGFβ inhibitor (e.g., SB431542), growth factors (e.g., EGF, bFGF). As an aspect, examples of the CDM medium include a medium to which one or more ingredients selected from albumin, BSA, HSA, FBS, and KSR, and TGFβ inhibitor and growth factors (e.g., EGF, bFGF).

The culture conditions and culture container in step (2) are compliant with step (1). The number of days of culturing in step (2), that is the number of days of expansion culturing is not particularly limited, but culturing may be performed for about 12 to 28 days, preferably about 14 to 22 days, and more preferably about 16 to 20 days. During the expansion culturing, for example, passaging may be performed with the interval of once per 3 to 5 days, and with the interval of once per 3 to 7 days, or once per 2 to 12 days.

Both the total number of cells and the proportion of the neural crest cells to the total number of cells in the cell population after expansion culturing in step (2) increase compared to the cell population before culturing. The total number of cells increases, for example, 4- to 30-fold and the proportion of neural crest cells to the total number of cells increases, for example, 1.2- to 5-fold.

### <Cell Population Containing Neural Crest Cell>

The cell population containing neural crest cells according to one embodiment is a cell population obtained by the method for producing neural crest cells. The cell population containing neural crest cells has the proportion of p75 positive cells (i.e., neural crest cells) to the total number of cells of 70% or more (e.g., 80% or more, 85% or more, 90% or more, or 95% or more), and has the proportion of neural crest progenitor cells to the total number of cells of 30% or less (e.g., 20% or less, 15% or less, 10% or less, or 5% or less).

In addition, the cell population after culturing in step (2) expresses at least one gene selected from TFAP2A, NGFR, EDN3, and TWIST1. These genes are neural crest cell markers, and it is possible to measure the gene expressions by a method common for those skilled in the art, such as PCR.

In the above, the neural crest cells are obtained by a method for inducing pluripotent stem cells, but the method for obtaining neural crest cells is not particularly limited, and for example, those obtained by separating from a living body can be used for a method for selectively culturing neural crest cells.

Also, provided is a method for culturing a cell population containing neural crest cells and/or neural crest progenitor cells, the method comprising a culturing step of adherent culturing the cell population containing the neural crest cell and/or the neural crest progenitor cell that are derived from a living body, in the presence of one or more extracellular matrices selected from the group consisting of a laminin with its α chain being an α1 chain and its β chain being a β1 chain, a laminin with its α chain being an α2 chain and its β chain being a β1 chain, a laminin with its α chain being an α2 chain and its β chain being a β2 chain, and a laminin with its α chain being an α5 chain and its β chain being a β1 chain, and extracellular matrices containing integrin-binding sites of these laminins. The culturing step in the culturing method is the same as that in step (2) described above.

The aforementioned cell population containing neural crest cells and/or neural crest progenitor cells derived from a living body is, for example, collected from a developing embryo. The cell population containing neural crest cells and/or neural crest progenitor cells collected from a developing embryo can be collected by sorting cell populations containing neural crest cells with a neural crest cell marker (SOX10, p75). The aforementioned developing embryo is, in a case of a human embryo, an embryo within 15 days of the development.

### [Method for Producing Mesenchymal Stem Cell]

A method for producing mesenchymal stem cells according to an embodiment includes the following step (3) and step (4):
(3) a step of producing a cell population containing neural crest cells by the aforementioned method for producing neural crest cells; and
(4) a step of culturing the cell population obtained in step (3) to induce differentiation into mesenchymal stem cells in the presence of bFGF.

Step (3) includes the above step (1) and step (2). Step (4) is a step of inducing differentiation of mesenchymal stem cells.

The mesenchymal stem cell (MSC) as used herein is one of stem cells present in an adult body and a cell having capacity of differentiating into a bone, cartilage, a blood vessel or a cardiomyocyte that are derived from a mesoderm. Examples of a mesenchymal cell marker include positive markers such as CD73, CD105, CD44, and negative markers such as CD45.

The medium in step (4) may be prepared by adding bFGF to the basal medium described in the above step (1), and it is preferable for the basal medium to be DMEM, RPMI-1640, and aMEM, and in particular, αMEM can be preferably used. The amount of bFGF to be added may be 0.01 to 1000 ng/ml, and it is preferable to be 0.1 to 100 ng/ml, or 1 to 30 ng/ml. Examples of ingredients to be arbitrary added to the medium include FBS and BSA.

The culture conditions and culture container in step (4) are compliant with steps (1) and (2). In order to facilitate engraftment of cells, the culture surface may be coated with the extracellular matrix. Examples of such an extracellular matrix include RetroNectin (recombinant human fibronectin fragment) and fibronectin, and in particular, fibronectin is preferably used. The amount to be coated may be 0.01 to 1000 µg/cm², and it is preferable to be 0.1 to 100 µg/cm², or 1 to 30 µg/cm², based on the culture surface area.

Culturing in step (4) may be conducted for about 4 to 20 days, and preferably about 8 to 16 days. The culturing duration can be decided depending on a desired cell differentiation level. The cell differentiation level can be decided from the measurement of the expression level of a differentiation marker. For example, it can be performed by increasing the expression level of CD73, CD 105 and CD44, or decreasing the expression level of CD45 using FACS, or by increasing a predetermined expression level of any one of CD73, CD 105 and CD44, or decreasing the expression level of CD45 using PCR.

A method for producing mesenchymal stem cells according to one embodiment includes the following step (3') and step (4'):
(3') a step of collecting a cell population containing neural crest cells and/or neural crest progenitor cells from an embryo; and
(4') a step of culturing the cell population obtained in step (3') to induce differentiation into mesenchymal stem cells in the presence of bFGF.

It is possible for the method for producing mesenchymal stem cells comprising the above step (3') and step (4') to apply the same aspect as the method for producing mesenchymal stem cells comprising the above step (3) and step (4).

### <Cell Population Containing Mesenchymal Stem Cell>

The cell population containing mesenchymal stem cell according to one embodiment is a cell population obtained by the aforementioned method for producing mesenchymal stem cell. The cell population containing mesenchymal stem cells has the proportion of CD45 negative cells, and CD73, CD105, and CD44 positive cells of 90% or more, to the total number of cells.

### [Therapeutic Agent and Pharmaceutical Composition]

The therapeutic agent or pharmaceutical composition according to one embodiment is a therapeutic agent or pharmaceutical composition containing the cell population of the mesenchymal stem cells or the cell population obtained by the aforementioned method for producing mesenchymal stem cell as an active ingredient. Specifically, it is a therapeutic agent for a disease selected from graft-versus-host disease (GvHD), acute respiratory distress syndrome (ARDS), asthma, serious heart failure , myocardial infarction, cerebral infarction, traumatic brain injury, brain tumor, spinal cord injury, critical limb ischemia, diabetic foot ulcer, hepatic cirrhosis, acute liver failure, chronic liver failure, Crohn's disease, sepsis, viral infection, epidermolysis bullosa, diabetes, diabetic organ dysfunction, atopic dermatitis, hypersensitivity, severe combined immunodeficiency syndrome, multiple myeloma, Kawasaki disease, scleroderma, alopecia, autoimmune hepatitis, lupus nephritis, aplastic anemia, rheumatoid arthritis, systemic lupus erythematosus, Sjogren's syndrome, psoriasis, hip osteoarthritis, knee osteoarthritis (OA), intervertebral disc degeneration, complicated skin and skin soft tissue infection, bacterial pneumonia, viral pneumonia, Pseudomonas aeruginosa infection, acquired immunodeficiency syndrome, rabies, influenza, osteonecrosis, osteopenia, alveolar bone loss, bone injury, osteogenesis imperfecta, spondylosyndesis, muscular atrophy, tendon injury, knee injury, muscle injury, musculoskeletal injury, osteoporosis, movement disorder disease, multiple sclerosis, cerebral palsy, dementia, optic neuritis, carpal tunnel syndrome, striatonigral degeneration, olivopontocerebellar atrophy, Tourette syndrome, facial hemiatrophy, periventricular leukomalacia, spinocerebellar ataxia, schizophrenia, bipolar disorder, major depressive disorder, anxiety disorder, adjustment disorder, alcoholism, congenital dysautonomia, encephalitis, epilepsy, spina bifida, amyotrophic lateral sclerosis, spinal muscular atrophy, primary lateral sclerosis, muscular dystrophy, Alzheimer's disease, Parkinson's disease, Fabry disease, Huntington's disease, mucopolysaccharidosis type I, Charcot-Marie-Tooth disease, neuropathic pain, peripheral neuropathy, trigeminal neuralgia, diabetic neuropathy, sciatica, spinal stenosis, hypoxic-ischemic encephalopathy, cerebral hemorrhage, breast cancer, metastatic pancreatic cancer, pancreatic ductal adenocarcinoma, gastrointestinal cancer, head and neck cancer, oral cancer, mesothelioma, metastatic non-small-cell lung cancer, melanoma, skin cancer, glioblastoma, solid cancer, adenocarcinoma, glioma, medulloblastoma, uveal melanoma, bronchiectasis, chronic bronchus disease, respiratory distress syndrome, idiopathic pulmonary fibrosis, pulmonary malformation, pulmonary emphysema, pneumoconiosis, respiratory failure, acute lung injury, lung injury, preeclampsia, congestive heart failure, angina pectoris, myocarditis, congenital heart disease, dilated cardiomyopathy, multiple organ failure, coronary artery disease, ischemia, varicose ulcer, thromboangiitis obliterans, peripheral arterial occlusive disease, intermittent claudication, pulmonary stenosis, arteriovenous fistula, peripheral vascular disease, angiogenic disorder, atherosclerosis, corneal injury, keratitis, corneal dystrophy, corneal ulcer, diabetic retinopathy, glaucoma, retinitis pigmentosa, dry age-related macular degeneration, xerophthalmia, corneal edema, wet age-related macular degeneration, fistula, wound healing, obesity, Pitt-Hopkins syndrome, pressure ulcer, leg ulcer, skin ulcer, insulin-dependent diabetes mellitus, non-insulin-dependent diabetes mellitus, hearing loss, perianal fistula, rectal fistula, anal fistula, rectovaginal fistula, alcoholic liver disease, pancreatitis, biliary atresia, gastrointestinal bleeding, primary sclerosing cholangitis, proctitis, esophageal injury, liver injury, inflammatory bowel disease, ulcerative colitis, premature ovarian failure, ovarian cancer, oligospermia, testicular disease, erectile dysfunction, penile induration, uterine injury, renal failure, diabetic nephropathy, renal injury, renal fibrosis, interstitial cystitis, IgA nephropathy, stress urinary incontinence, urge urinary incontinence , C3 glomerulopathy, thalassemia, leukemia, systemic inflammatory response syndrome, radiation sickness, skin burn injury, reperfusion injury, syndrome X, metabolic disease, oral mucositis, periodontal disease, gum disease, and autism spectrum disorder; or a pharmaceutical composition for applied use selected from suppression of rejection associated with transplantation in regenerative therapy, support for transplantation (organ transplantation, hematopoietic stem cell transplantation, bone marrow transplantation, corneal transplantation, islet transplantation), improvement in aging, and use for vaccine and the like.

The effective amount of the active ingredient in the therapeutic agent or pharmaceutical composition varies depending on purposes of administration, administration methods, and conditions of the subject to be administered (gender, age, body weight, disease state and the like), but for example, it is preferable that 1.0 × 10⁸ cells or more of the effective amount of the cell population be used based on the number of cells of the mesenchymal stem cells.

The therapeutic agent or pharmaceutical composition according to one embodiment may contain a pharmaceutically acceptable carrier, in addition to the effective amount of the active ingredient described above. It is possible to use a physiological aqueous solvent (e.g., physiological saline, buffer solution, or serum-free medium) as the pharmaceutically acceptable carrier. The therapeutic agent or pharmaceutical composition can contain commonly used preservative, stabilizer, reducing agent, isotonic agent or the like in medicines containing tissues or cells to be transplanted in a transplantation therapy, as necessary.

It is possible to produce a cell population as a cell suspension solution by suspending it in an appropriate physiological aqueous solvent. If necessary, the cell population can be cryopreserved by adding a cryopreserving agent, thawed when in use, washed with a buffer solution, and used for a transplantation therapy.

When the cell population of the active ingredient is produced from established pluripotent stem cells, it is possible to mass produce a cell population of stable quality to use in transplantation, by identifying it by a marker or the like and performing quality control. In addition, it is possible to preserve the cell population, and therefore, it is possible to prepare the cell population according to the time of transplantation for a patient.

### [Method for Determining Progress State of Differentiation of Pluripotent Stem Cell]

The method for determining a progress state of differentiation of pluripotent stem cells when induing differentiation of the pluripotent stem cells into neural crest cells and/or neural crest progenitor cells includes the following steps (I) to (IV):
(I) a step of collecting a part of a cell population during culturing at a desired time point;
(II) a step of measuring the expression level of a predetermined gene in the collected cell population;
(III) a step of comparing the expression level of the measured gene with a threshold; and
(IV) a step of determining that when the expression level of the measured gene is equal to or higher than the threshold, the cultured cell population at the desired time point is capable of differentiating into the neural crest cell and/or the neural crest progenitor cell.

### <Step (I) Step of Collecting Part of Cell Population>

In step (I), the term "desired time point" means any time point in the culturing for inducing differentiation of the pluripotent stem cells into neural crest cells and/or neural crest progenitor cells. Here, the culturing for inducing differentiation of the pluripotent stem cells into neural crest cells and/or neural crest progenitor cells may include steps (1) and (2) in the method for producing a cell population containing the pluripotent stem cells. The desired time point in step (I) may be, for example, any time point in the step of inducing differentiation of pluripotent stem cells into neural crest cells and/or neural crest progenitor cells to obtain a cell population containing the neural crest cells and/or the neural crest progenitor cells (in step (1)), or may be any time point in the step of selectively culturing neural crest cells from the cell population containing neural crest cells and/or neural crest progenitor cells (in step (2)).

The cell population during culturing in step (I) may be any cell population during culturing in the culture for inducing differentiation of pluripotent stem cells into neural crest cells and/or neural crest progenitor cells, and for example, may be the cell population during culturing in step (1), or may be the cell population during culturing in step (2). Collecting a part of the cell population can be performed by a conventional method, centrifugation or the like, and further, the number of cells to be collected is not particularly limited as long as the number of cells is sufficient to measure the expression level of gene described later.

### <Step (II) Step of Measuring Expression Level of Predetermined Gene>

The predetermined gene may be any gene as long as it can confirm the differentiation of pluripotent stem cells into neural crest cells and/or neural crest progenitor cells, and may be any gene of which expression level increases in the cell population after inducing differentiation of pluripotent stem cells into neural crest cells and/or neural crest progenitor cells, and it is preferable that it be a gene that rarely expresses in pluripotent stem cells, but specifically expresses in neural crest cells and/or neural crest progenitor cells. The gene of which expression level increases in the cell population after (the step of) inducing differentiation of pluripotent stem cells into neural crest cells and/or neural crest progenitor cells may be a gene that has enhanced expression after (the step of) inducing the differentiation, or may be a gene that has enhanced during an expansion culturing period. Here, the term "gene that has enhanced expression after (the step of) inducing the differentiation" means a gene that has enhanced expression compared to the pluripotent stem cells at the completion of (the step of) inducing the differentiation, and it includes a gene that has gradually enhanced expression along with the progress of the induction of differentiation. In addition, "gene that has enhanced expression after (the step of) inducing the differentiation" includes a gene that has enhanced expression during an expansion culturing period. The predetermined gene may include, for example, one or more genes that has enhanced expression after (the step of) inducing the differentiation, and/or one or more genes that has enhanced expression during an expansion culturing period. The predetermined gene may be one combined with a undifferentiation marker gene. The expression level of a gene as used herein means the amount of expressed product of the gene. The expressed product of the gene may be, for example, a mRNA of the gene, or may be a protein or polypeptide that the gene encodes.

The method for measuring the expression level of a gene is not particularly limited, and it can be performed by a method known to those skilled in the art. Examples of such a method include reverse transcription-polymerase chain reaction (RT-PCR), real-time RT-PCR, Northern blot, RNA sequencing, microarray, Western blotting, radioimmunoassay, and ELISA.

The one or more genes that has enhanced expression after (the step of) inducing the differentiation may be, for example, SOX10, RHOB, FOXD3, and NOTCH1. The one or more genes that has enhanced expression during the expansion culturing period may include one or more selected from the group consisting of, for example, SOX9, TFAP2A, NGFR, TWIST1, and EDN3, and it is preferable to be TWIST1 and/or NGFR. Note that SOX9, TFAP2A, NGFR, TWIST1, and EDN3 mentioned above are included in the genes that has enhanced expression after (the step of) inducing the differentiation. According to the present inventors' experiments (Examples 4 to 6), these genes are suggested to be genes that specifically express in neural crest cells and/or neural crest progenitor cells. Examples of the undifferentiation marker gene include NANOG and Oct3/4.

### <Step (III) Step of Comparing Expression Level of Measured Gene with Threshold>

In step (III), the expression level of the gene measured in step (II) above is compared with a preset threshold. The threshold in step (III) may be set based on the expression level of the predetermined gene in pluripotent stem cells before differentiation-induction, or may be set based on the expression level of the predetermined gene in the cultured cell population which has not reached a desired progress state of the differentiation. Here, the threshold may vary depending on the expressed products of the genes (mRNA or polypeptide).

### <Step (IV) Step of Determining Whether Above Cell Population Is Capable Of Differentiating into Neural Crest Cell and/or Neural Crest Progenitor Cell>

Since the predetermined gene is a gene of which expression level increases in the cell population after inducing differentiation of pluripotent stem cells into neural crest cells and/or neural crest progenitor cells (gene that has enhanced expression after (the step of) inducing the differentiation and/or genes that has enhanced expression during the expansion culturing period), and the threshold is an indicator for a cultured cell population which has not reached a desired progress state of the differentiation, when the expression level of the gene is higher than the threshold, it is possible to determine that the cultured cell population at the desired time point is capable of differentiating into neural crest cells and/or neural crest progenitor cells.

The method for determining a progress state of differentiation of pluripotent stem cells according to one embodiment can be understood to be a method for screening for a substance capable of differentiating the pluripotent stem cell population into neural crest cell and/or neural crest progenitor cell.

By the method above, the cell population determined as capable of differentiating into neural crest cells and/or neural crest progenitor cells may be collected, and further the neural crest cells in the cell population may be selectively cultured. By culturing the cell population containing thus obtained neural crest cells according to the method for producing the cell population containing mesenchymal stem cells, the cell population containing mesenchymal stem cells can be produced.

[Use of Extracellular Matrix in Neural Crest Cell] An aspect of the present invention provides use of one or more extracellular matrices selected from the group consisting of laminin with its α chain being an α1 chain and its β chain being a β1 chain, laminin with its α chain being an α2 chain and its β chain being a β1 chain, laminin with its α chain being an α2 chain and its β chain being a β2 chain, and laminin with its α chain being an α5 chain and its β chain being a β1 chain, and extracellular matrices containing integrin-binding sites of these laminins, in selective culturing of neural crest cells in a cell population containing neural crest cells and/or a neural crest progenitor cells.

The present invention will be described in more detail using Examples described below; however, the present invention is not limited to these.

### EXAMPLES

### [Example 1: Inducing Differentiation into Neural Crest Cells and Expansion Culturing Using iMatrix-221]

### <Culturing of iPS Cell>

As the iPS cell, used was an iPS cell (SMCB004) established from an adult peripheral blood mononuclear cell using Cyto Tune (trademark)-2.0 (ID Pharma Co., Ltd.). The E8 fragment of laminin 511, iMatrix-511, (Nippi, Incorporated, Japan) was added to a 6-well plate to be 0.5 µg/cm², the mixture was left stand still in an incubator at 37°C for an hour, added with a trace amount of a medium followed by removing the supernatant by suction, and then washed with PBS to obtain a plate coated with iMatrix-511. Next, iPS cells suspended in StemFit (registered trademark) AK03N (Ajinomoto Co., Inc., Japan) medium was seeded at 8000 cells/well, and cultured in a 5% CO₂ incubator at 37°C for 5 days. Note that, in this Example, the day of seeding iPS cells is designated Day 0, and the experiment days thereafter is represented as the number of days from the day. For example, 5 days after culturing iPS cells is represented as Day 5.

### <Inducing Differentiation of iPS Cells into Neural Crest Cells>

Five days after iPS cell seeding (Day 5), substitution was performed with a neural crest cell inducing medium obtained by adding, to CDM medium (IMDM/Ham's F-12 1:1 (GIBCO, USA), 1× Chemically Defined Lipid Concentrate (GIBCO), 15 µg/ml of Optiferrin (InVitria, Inc., USA), 7 µg/mL of insulin (Sigma-Aldrich Corp., USA), and 450 µM of monothioglycerol (FUJIFILM Wako Pure Chemical Corporation, Japan)), 20% KSR (GIBCO), 10 µM of SB431542 (FUJIFILM Wako Pure Chemical Corporation) and 1 µM of CHIR99021 (FUJIFILM Wako Pure Chemical Corporation), and the mixture was cultured in a 5% CO₂ incubator at 37°C for 9 days to induce differentiation into neural crest cells. In this Example, this differentiation-induction time point is referred to as passage 0 (P0).

### <Expansion (Selectively) Culturing Neural Crest Cell>

The E8 fragment of laminin 221, iMatrix-221, (Nippi, Incorporated) was added to a 6-well plate to be 0.5 µg/cm², the mixture was left stand still in an incubator at 37°C for an hour, added with a trace amount of a medium followed by removing the supernatant by suction, and then washed with PBS to obtain an iMatrix-221-coated plate.

Next, at Day 14, cells that had been cultured by inducing differentiation into neural crest cells for 9 days were collected by peeling off using a trypsin/EDTA solution, single-cell-suspended, and cells suspended in a neural crest cell-expansion culturing medium (medium obtained by adding 5 mg/mL of BSA (Proliant Biologicals, Inc., USA), 10 µM of SB431542, 10 ng/mL of bFGF (FUJIFILM Wako Pure Chemical Corporation) and 20 ng/mL of EGF (R&D Systems, Minneapolis, USA) to a CDM medium) were seeded on the iMatrix-221-coated plate at 50000 cells/well, and cultured in a 5% CO₂ incubator at 37°C for 10 days. In this Example, this expansion culturing, in other words, the expansion culturing after one passage is referred to as passage 1 (P1).

On 10 days after the seeding (Day 24), the first passage was performed. Cells were collected by peeling off using a trypsin/EDTA solution, cells suspended in a neural crest cell-expansion culturing medium were seeded on the iMatrix-221-coated plate at 50000 cells/well, and cultured in a 5% CO₂ incubator at 37°C. In this Example, this expansion culture, in other words, the expansion culture after two passages is referred to as passage 2 (P2). Thereafter, passages were performed once every 3 to 7 days (e.g., P3 or P4), and passage processing was further performed several times.

### <Confirmation of Result of Differentiation-Induction and Expansion Culturing By Flow Cytometry>

The iPS cells (undifferentiated iPS cells) before seeding (Day 0), and the cells that had been cultured by inducing differentiation into neural crest cells (NCC) for 9 days (P0: Day 14), and the cells after expansion culturing P1 (Day 24), P2 (Day 32) and P3 (Day 35) were collected by peeling off, single-cell-suspended, and then dyed with a CD271 antibody for FACS (BD Biosciences, Inc.), which is an antibody with neural crest cell marker p75 as an antigen, and confirmed by FACS (BD Accuri (BD Biosciences, Inc., USA)).

The results are shown in Figure 1. It can be seen from Figure 1 that the proportion of p75 positive cells (p75 positive cell ratio) in the cell population after inducing differentiation into neural crest cells (P0) was 52.5%, and the p75 positive cell ratio after expansion culturing after one passage (P1) was 71.4%, the p75 positive cell ratio after expansion culturing after two passages (P2) was 97.9%, and further the p75 positive cell ratio after expansion culturing after three passages (P3) was 98.3%. From this, it was found that p75 positive cells (i.e., neural crest cells) were selectively cultured by the expansion culturing and enriched. It was confirmed that it was possible to obtain 98% or more of p75 positive cell ratios by only the expansion culturing method of the present invention, without sorting the p75 positive cells from the cell population after inducing differentiation into neural crest cells (P0). Further, it was possible to obtain 98% or more of p75 positive cell ratios by passaging of other iPS cells (201B7 strain).

### <Inducing Differentiation into Mesenchymal Stem Cell>

Fibronectin (Sigma-Aldrich Corp) was added to a plate to be 10 µg/cm², and the mixture was left stand still in an incubator at 37°C for an hour to obtain a fibronectin-coated plate.

Next, at Day 32 (P2), the expansion cultured neural crest cells were collected by peeling off using a trypsin/EDTA solution, single-cell-suspended, and cells suspended in a neural crest cell-expansion culturing medium were seeded on the fibronectin-coated plate, and cultured in a 5% CO₂ incubator at 37°C. On the next day, the mixture was converted to a mesenchymal stem cell medium (medium obtained by adding 10% FBS (GIBCO) and 10 mg/mL of bFGF (FUJIFILM Wako Pure Chemical Corporation) to an α-MEM (Nacalai Tesque, Inc., Japan)), and cultured in a 5% CO₂ at 37°C to induce differentiation into mesenchymal stem cells.

Three days after the initiation of inducing differentiation into mesenchymal stem cells (Day 34), the cells were collected by peeling off using a trypsin/EDTA solution, single-cell-suspended, and cultured in the mesenchymal stem cell medium in a 5% CO₂ at 37°C, and then, passaged when being confluent.

Further, after inducing differentiation into mesenchymal stem cells, the cell population after culture passaging for 9 days (Day 40) was collected by peeling off, single-cell-suspended, and then dyed with a CD73, CD105, CD44 and CD45 antibodies for FACS (BD Biosciences, Inc.), which are mesenchymal stem cell markers, and confirmed by FACS in the same manner as above. The results are shown in Figure 2. It can be seen from Figure 2 that the differentiation info mesenchymal stem cells was succeeded. Note that it was confirmed that it was possible to induce differentiation into at least osteoblast, chondrocyte and adipocyte from mesenchymal stem cells that were obtained by inducing differentiation of other iPS cell lines in the same manner.

### [Example 2: Expansion Culturing Using Laminin 211]

Inducing differentiation of iPS cells into neural crest cells was conducted in a manner similar to that in Example 1. The cell population after inducing differentiation was expansion culturing by the following method.

Laminin 211 (BioLamina AB) was added to a 6-well plate to be 0.45 µg/cm², the mixture was left stand still in an incubator at 37°C for an hour, added with a trace amount of a medium followed by removing the supernatant by suction, and then washed with PBS to obtain a Laminin-211-coated plate.

Next, at Day 14, the cells cultured by inducing differentiation into neural crest cells for 9 days were collected by peeling off using a trypsin/EDTA solution, single-cell-suspended, seeded with a neural crest cell-expansion culturing medium, and cultured in a 5% CO₂ incubator at 37°C for 10 days.

### <Comparison of the Results of Example 1 and Example 2>

The cells after expansion culturing for 10 days (P1) of Example 1 and Example 2 were observed by an optical microscope, and the results thereof are shown in Figure 3. In Figure 3, (A) and (B) are images of the cells after expansion culturing for 10 days of Example 2, and (C) and (D) are images of the cells after expansion culturing for 10 days of Example 1. As can be seen in Figure 3, the cells in Example 1 were generally spread out and increased, whereas the cells in Example 2 were dense. It was suggested that the expansion culturing method of Example 1 was more suitable for proliferation of neural crest cells. In addition, since the density states of the cells differed, it was also suggested that there was a possibility that laminin 221 and laminin 211 play a different role concerning the cell engraftment.

### [Example 3: Expansion Culturing Using Various Extracellular Matrices]

Inducing differentiation of iPS cells into neural crest cells was conducted in a manner similar to that in Example 1.

Fibronectin (Sigma-Aldrich Corp) was added to a plate to be 10 µg/cm², and the mixture was left stand still in an incubator at 37°C for an hour to obtain a fibronectin-coated plate. In addition, according to the description in Example 1, Laminin-111 (BioLamina AB), Laminin-211 (BioLamina AB) and Laminin-521 (BioLamina AB) were each added to a 6-well plate to be 0.5 µg/cm², the mixture was left stand still in an incubator at 37°C for an hour, added with a trace amount of a medium followed by removing the supernatant by suction, and then washed with PBS to obtain a Laminin-111-coated plate, a Laminin-211-coated plate, and a Laminin-521-coated plate. Further, the E8 fragments, iMatrix-511, (Nippi, Incorporated) and iMatrix-221 (Nippi, Incorporated), were added to a 6-well plate to be 0.5 µg/cm², the mixture was left stand still in an incubator at 37°C for an hour, added with a trace amount of a medium followed by removing the supernatant by suction, and then washed with PBS to obtain a iMatrix-511 -coated plate and a iMatrix-221-coated plate.

Next, at Day 14 (P0), the cells cultured by inducing differentiation into neural crest cells for 9 days were collected by peeling off using a trypsin/EDTA solution, single-cell-suspended, and seeded with a neural crest cell-expansion culturing medium at 50000 cells/well on the respective coated plates, and expansion cultured in a 5% CO₂ incubator at 37°C. On the day following the seeding, cell states of adhering to various extracellular matrices were observed by an optical microscope, and the results thereof are shown in Figure 4. As can be seen in Figure 4, it was found that the states of adhering to a different extracellular matrix differed despite of the same cell population.

Thereafter, passages were performed on Day 23, Day 26, Day 29, Day 32 and Day 35 in the same manner as above, and the expansion culturing for P1, P2, P3, P4, and P5 was conducted on each of them. In the same manner as in Example 1, cells of P0, P1, P2, P3, P4, and P5 were collected by peeling off, single-cell-suspended, and then the expression of the neural crest cell marker p75 was confirmed by flow cytometry. The results of the proportion of p75 expressing cells (p75 positive cells) to the total number of cells (p75 positive cell ratio) are shown in Figure 5. However, there were results only up to P4 in the Laminin-521 coating experiment. As can be seen in Figure 5, it was confirmed that the p75 positive cell ratios in the cell populations that were expansion cultured on the coating of Laminin-111, iMatrix-211, iMatrix-511, and iMatrix-221 increased by continuation of expansion culturing, among various extracellular matrices. In addition, it was confirmed that 90% or more of p75 positive cell ratios was obtained by the expansion culturing using these extracellular matrices. In other words, it is possible to use the cell population obtained by expansion culturing using these extracellular matrices as it is for inducing differentiation into mesenchymal stem cells, without sorting the p75 positive cells (i.e., neural crest cells).

Further, the gene expressions of NGFR, TFAP2A, TWIST1, SOX10 and SOX9 in the iPS cells before seeding (Day 0), the differentiation-induced neural crest cells (P0), and the cell populations of P1, P2, P3, P4, and P5 expansion cultured using various extracellular matrices were confirmed by real-time PCR (Thermo Fisher, Inc.). The primer sequences used were as follows. In addition, β-actin was used as internal standard.

**[Table 1]**

| | Forward primer | Reverse primer |
|---|---|---|
| NGFR | CCGTTGGATTACACGGTCCA (SEQ ID NO: 1) | GACAGGGATGAGGTTGTCGG (SEQ ID NO: 2) |
| TFAP2A | AGGGCCTCGGTGAGATAGTT (SEQ ID NO: 3) | AAGAGTTCACCGACCTGCTG (SEQ ID NO: 4) |
| TWIST1 | TTTTAAAAGTGCGCCCCACG (SEQ ID NO: 5) | TCTCGGTCTGGAGGATGGAG (SEQ ID NO: 6) |
| SOX10 | GAGCTGGACCGCACACCTTGGG (SEQ ID NO: 7) | AACGCCCACCTCCTCGGACCTC (SEQ ID NO: 8) |
| SOX9 | GAGCAGACGCACATCTC (SEQ ID NO: 9) | CCTGGGATTGCCCCGA (SEQ ID NO: 10) |

The respective expression levels of the genes measured by real-time PCR was corrected with the expression level of β-actin, and further, the relative expression levels of the respective genes with the expression level of iPS cells at Day 0 to be "1" are shown in Figures 6 and 7. In each grape, Day 0, P0, P1, P2, P3, P4, and P5 are shown in this order from left. However, there were results only up to P4 in the Laminin-521 coating experiment. It was confirmed that, comparing with P0 time point, the expressions of NGFR, TFAP2A, and TWIST1, which are neural crest cell markers, increased after P1 by the continuation of expansion culturing in the cell population that was cultured on Laminin-111, iMatrix-211, iMatrix-511, and iMatrix-221 in which 90% or more of p75 positive cell ratios was able to be obtained by flow cytometry analysis (Figure 6).

As shown in Figures 6 and 7, the cell population containing neural crest cells, obtained by the culturing step (in particular, expansion culturing including multiple time passages) expressed more than the neural crest cells (P0) undifferentiation-induced by at least one of TFAP2A, NGFR, and TWIST1. From this, it was confirmed that the cells obtained by expansion culturing using the extracellular matrices are neural crest cells from the viewpoint of gene expression profiling.

Further, it was confirmed that the expression level of SOX9 genes, which is essential for adherent culturing, increased in the cell population expansion cultured by the extracellular matrices. In addition, from the expression state of SOX9 genes, it was suggested that it was suitable as an expansion culturing method by adherent culturing (Figure 7). In Example 3, since the cell population containing neural crest cells selectively cultured is a cell population in which the expression level of SOX10 genes decreases and SOX9 highly expresses, the culturing conditions used in the Example has a tendency of exhibiting profiling that decreases the expression of SOX10 genes and increases the expression of SOX9 genes. In other words, the cell population after a culturing step of adherent culturing the cell population containing neural crest cells and/or neural crest progenitor cells has characteristics of decreasing the expression of SOX10 genes and increasing the expression of SOX9 genes. Specifically, comparing to the cell population at the time of the initiation of expansion culturing, the cell population in which the expression level of SOX10 genes decreases and the expression level of SOX9 genes increases is an aspect of the present invention.

### [Example 4: Cluster Analysis Based on Expression Level of Gene at the Time of Expansion Culturing Using Various Extracellular Matrices]

The expression levels of genes of P1 to P5 cells produced by expansion culturing using various extracellular matrices in Example 3 were acquired by micro array analysis. Micro array analysis was conducted by using GeneChip (registered trademark) Human Genome U133 Plus 2.0 Array (Applied Biosystems, Inc.). Next, cluster analysis for the respective genes were conducted by Ward's method using R, based on the comparison of the signal intensities of each passage (P1 to P5) from the expression levels of the entire genes obtained by micro array analysis. The Ward's method is one of hierarchical clustering approaches, and an approach to extracting hierarchical structures in cluster from data. The Ward's method binds data so that the dispersion is minimal, and performs clustering. The results of cluster analysis by the Ward's method are shown in Figure 8.

As shown in Figure 8, the results from the cluster analysis caused the cell to be clearly categorized with the expression states of genes by the differences of extracellular matrices, similar to the differences of the expression states of p75 by flow cytometry shown in Figure 5. From the above results, it was revealed that the expression states of neural crest cell-associated genes and undifferentiated marker genes differed between the extracellular matrix group of iMatrix-221, iMatrix-511, Laminin-211, and Laminin-111, and the extracellular matrix group of Laminin-521 and Fibronectin which are different in the expression states of p75. In addition, since the cell was clearly categorized with the expression states of genes from the differences of extracellular matrices, it was suggested that neural crest cells in the expression states of gene with a certain tendency (neural crest cells in the cell state with a certain tendency) can be obtained in the same extracellular matrix group. The expression states of gene with a certain tendency is that the cells obtained by culturing exhibit a tendency (measurable pattern of gene expression) in the gene expression.

### [Example 5: Comparison of Expression of Each Gene at the Time of Expansion Culturing Using Various Extracellular Matrices]

Tables 2 and 3 show that undifferentiated gene markers and neural crest cell-associated gene data were extracted from the expression level of the genes obtained in Example 4. Shown are the results of comparing signal intensities between iPS cells and P0 or P5 on the relative expression levels of respective genes of extracted undifferentiated marker genes and neural crest cell-associated gene.

**[Table 2]**

| Gene name | iPS cell | P0 | P1 | P2 | P3 | P4 | P5 |
|---|---|---|---|---|---|---|---|
| NANOG | 100% | ≤1% | ≤1% | ≤1% | ≤1% | ≤1% | ≤1% |
| Oct3/4 | 100% | ≤1% | ≤1% | ≤1% | ≤1% | ≤1% | ≤1% |

**[Table 3]**

| Gene name | iPS cell | P0 | Laminin-111 P5 | Laminin-211 P5 | iMatrix-221 P5 | iMatrix-511 P5 |
|---|---|---|---|---|---|---|
| SOX10 | 1 | 27.0 | 0.2 | 0.2 | 0.4 | 0.3 |
| RHOB | 1 | 32.0 | 3.6 | 3.5 | 3.8 | 4.1 |
| FOXD3 | 1 | 4.9 | 0.0 | 0.0 | 0.0 | 0.0 |
| NOTCH1 | 1 | 1.2 | 0.7 | 1.1 | 1.2 | 0.9 |
| SOX9 | 1 | 3.1 | 5.7 | 6.9 | 8.2 | 4.5 |
| TFAP2A | 1 | 85.0 | 144.0 | 140.0 | 153.0 | 148.0 |
| NGFR | 1 | 16.0 | 57.0 | 57.0 | 38.0 | 55.0 |
| TWIST1 | 1 | 90.0 | 434.0 | 401.0 | 438.0 | 381.0 |
| EDN3 | 1 | 4.2 | 141.0 | 131.0 | 161.0 | 108.0 |

As shown in Table 2, it is found that the undifferentiated marker genes (NANOG, and Oct3/4) highly expressed in the iPS cells, but expressed almost none after P1, and the remaining of the undifferentiated iPS cells was at the minimum in inducing into neural crest cells. Further, as shown in Table 3, in the neural crest cell-associated genes, there are a gene group that has enhanced expression after inducing into neural crest cells (up to P0) (SOX10, RHOB, FOXD3, and NOTCH1) and a gene group that has enhanced expression at a later stage of induction (P1 to P5) (SOX9, TFAP2A, NGFR, TWIST1, and EDN3), and they exhibited similar behavior even though the extracellular matrices are different.

### [Example 6: Comparison of Expression States of Genes When Inducing Differentiation into Neural Crest Cells Using Extracellular Matrices in Which Only β Chains Are Different]

From the results of cluster analysis in Example 4, it was revealed that the expression states of, in particular, neural crest cell-associated genes and undifferentiated marker genes changed by altering extracellular matrices from P0 and culturing. Next, for the purpose of examination in detail, cluster analysis was conducted on the P0 to P5 cells based on the entire gene expression obtained by micro array analysis, using the extracellular matrices in which only β chains are different. Cluster analysis was conducted in the same manner as in Example 4 except that Laminin-211 and iMatrix- 221 were used as the extracellular matrices in which only β chains are different. The results thereof are shown in Figure 9.

As shown in Figure 9, it was suggested that the cells were neural crest cells but their expression states were different, and cell states of the neural crest cells differed, as a result of inducing differentiation of iPS cells into neural crest cells on Laminin-211 and iMatrix-221 in which only β chains are different.

### Industrial Applicability

According to the method for producing a neural crest cell of the present invention, it is possible to stably mass produce the neural crest cell from a pluripotent stem cell, and as a result, it is possible to stably mass produce a mesenchymal stem cell from the pluripotent stem cell.

## Claims

1. A method for selectively culturing a neural crest cell in a cell population comprising a neural crest cell and/or a neural crest progenitor cell, the method comprising:
a culturing step of adherent culturing the cell population comprising the neural crest cell and/or the neural crest progenitor cell, in the presence of one or more extracellular matrices selected from the group consisting of a laminin with its α chain being an α1 chain and its β chain being a β1 chain, a laminin with its α chain being an α2 chain and its β chain being a β1 chain, a laminin with its α chain being an α2 chain and its β chain being a β2 chain, and a laminin with its α chain being an α5 chain and its β chain being a β1 chain, and extracellular matrices comprising integrin-binding sites of these laminins.

2. The culturing method according to claim 1, wherein a γ chain of the laminin is a γ1 chain.

3. The culturing method according to claim 1 or 2, wherein the laminin is laminin 221.

4. The culturing method according to any one of claims 1 to 3, wherein the integrin-binding sites of the laminins include an E8 fragment of the laminin.

5. The culturing method according to any one of claims 1 to 4, wherein the cell population after culturing in the culturing step expresses at least one gene selected from EDN3, TFAP2A, NGFR and TWIST1.

6. The culturing method according to any one of claims 1 to 5, wherein at least a part of a culture surface with the cell population adhered thereon is coated with the extracellular matrix, and the cell population is adherent cultured in a medium in the culturing step.

7. The culturing method according to claim 6, wherein the medium is a medium suitable for maintaining the neural crest cell.

8. The culturing method according to any one of claims 1 to 7, wherein both a total number of cells and a proportion of the neural crest cell to the total number of cells in the cell population after culturing increase compared to the cell population before culturing.

9. A method for producing a cell population comprising a neural crest cell, the method comprising:
(1) a step of inducing differentiation of a pluripotent stem cell into a neural crest cell and/or a neural crest progenitor cell to obtain a cell population comprising the neural crest cell and/or the neural crest progenitor cell; and
(2) a step of adherent culturing the cell population obtained in step (1) by the culturing method according to any one of claims 1 to 8.

10. The production method according to claim 9, wherein the cell population obtained in step (1) is subjected to step (2) without being sorted or selected with a neural crest cell marker.

11. The production method according to claim 9 or 10, wherein step (1) comprises culturing a cell population comprising a pluripotent stem cell in a medium comprising at least one SMAD signaling inhibitor and at least one Wnt signaling activator.

12. The production method according to claim 11, wherein the SMAD signaling inhibitor is a TGFβ inhibitor.

13. The production method according to claim 11 or 12, wherein the Wnt signaling activator is a GSK3β inhibitor.

14. The production method according to any one of claims 9 to 13, wherein step (1) is conducted for 7 to 18 days.

15. The production method according to any one of claims 9 to 14, wherein the medium in step (1) is a chemically defined medium without serum replacement or without serum.

16. A method for producing a cell population comprising a mesenchymal stem cell, the method comprising:
(3) a step of producing a cell population comprising a neural crest cell by the production method according to any one of claims 9 to 15; and
(4) a step of culturing the cell population obtained in step (3) to induce differentiation into a mesenchymal stem cell in the presence of bFGF.

17. A cell population comprising a mesenchymal stem cell, produced by the production method according to claim 16.

18. A therapeutic agent for a disease selected from graft-versus-host disease (GvHD), acute respiratory distress syndrome (ARDS), asthma, serious heart failure, myocardial infarction, cerebral infarction, traumatic brain injury, brain tumor, spinal cord injury, critical limb ischemia, diabetic foot ulcer, hepatic cirrhosis, acute liver failure, chronic liver failure, Crohn's disease, sepsis, viral infection, epidermolysis bullosa, diabetes, diabetic organ dysfunction, atopic dermatitis, hypersensitivity, severe combined immunodeficiency syndrome, multiple myeloma, Kawasaki disease, scleroderma, alopecia, autoimmune hepatitis, lupus nephritis, aplastic anemia, rheumatoid arthritis, systemic lupus erythematosus, Sjogren's syndrome, psoriasis, hip osteoarthritis, knee osteoarthritis (OA), intervertebral disc degeneration, complicated skin and skin soft tissue infection, bacterial pneumonia, viral pneumonia, Pseudomonas aeruginosa infection, acquired immunodeficiency syndrome, rabies, influenza, osteonecrosis, osteopenia, alveolar bone loss, bone injury, osteogenesis imperfecta, spondylosyndesis, muscular atrophy, tendon injury, knee injury, muscle injury, musculoskeletal injury, osteoporosis, movement disorder disease, multiple sclerosis, cerebral palsy, dementia, optic neuritis, carpal tunnel syndrome, striatonigral degeneration, olivopontocerebellar atrophy, Tourette syndrome, facial hemiatrophy, periventricular leukomalacia, spinocerebellar ataxia, schizophrenia, bipolar disorder, major depressive disorder, anxiety disorder, adjustment disorder, alcoholism, congenital dysautonomia, encephalitis, epilepsy, spina bifida, amyotrophic lateral sclerosis, spinal muscular atrophy, primary lateral sclerosis, muscular dystrophy, Alzheimer's disease, Parkinson's disease, Fabry disease, Huntington's disease, mucopolysaccharidosis type I, Charcot-Marie-Tooth disease, neuropathic pain, peripheral neuropathy, trigeminal neuralgia, diabetic neuropathy, sciatica, spinal stenosis, hypoxic-ischemic encephalopathy, cerebral hemorrhage, breast cancer, metastatic pancreatic cancer, pancreatic ductal adenocarcinoma, gastrointestinal cancer, head and neck cancer, oral cancer, mesothelioma, metastatic non-small-cell lung cancer, melanoma, skin cancer, glioblastoma, solid cancer, adenocarcinoma, glioma, medulloblastoma, uveal melanoma, bronchiectasis, chronic Bronchus disease, respiratory distress syndrome, idiopathic pulmonary fibrosis, pulmonary malformation, pulmonary emphysema, pneumoconiosis, respiratory failure, acute lung injury, lung injury, preeclampsia, congestive heart failure, angina pectoris, myocarditis, congenital heart disease, dilated cardiomyopathy, multiple organ failure, coronary artery disease, ischemia, varicose ulcer, thromboangiitis obliterans, peripheral arterial occlusive disease, intermittent claudication, pulmonary stenosis, arteriovenous fistula, peripheral vascular disease, angiogenic disorder, atherosclerosis, corneal injury, keratitis, corneal dystrophy, corneal ulcer, diabetic retinopathy, glaucoma, retinitis pigmentosa, dry age-related macular degeneration, xerophthalmia, corneal edema, wet age-related macular degeneration, fistula, wound healing, obesity, Pitt-Hopkins syndrome, pressure ulcer, leg ulcer, skin ulcer, insulin-dependent diabetes mellitus, non-insulin-dependent diabetes mellitus, hearing loss, perianal fistula, rectal fistula, anal fistula, rectovaginal fistula, alcoholic liver disease, pancreatitis, biliary atresia, gastrointestinal bleeding, primary sclerosing cholangitis, proctitis, esophageal injury, liver injury, inflammatory bowel disease, ulcerative colitis, premature ovarian failure, ovarian cancer, oligospermia, testicular disease, erectile dysfunction, penile induration, uterine injury, renal failure, diabetic nephropathy, renal injury, renal fibrosis, interstitial cystitis, IgA nephropathy, stress urinary incontinence, urge urinary incontinence , C3 glomerulopathy, thalassemia, leukemia, systemic inflammatory response syndrome, radiation sickness, skin burn injury, reperfusion injury, syndrome X, metabolic disease, oral mucositis, periodontal disease, gum disease, and autism spectrum disorder; or a pharmaceutical composition for applied use selected from suppression of rejection associated with transplantation in regenerative therapy, support for transplantation (organ transplantation, hematopoietic stem cell transplantation, bone marrow transplantation, corneal transplantation, and islet transplantation), improvement in aging, and use for vaccine, the therapeutic agent or pharmaceutical composition comprising the cell population comprising the mesenchymal stem cell according to claim 17 as an active ingredient.

19. A method for determining a progress state of differentiation of a pluripotent stem cell when inducing differentiation of the pluripotent stem cell into a neural crest cell and/or a neural crest progenitor cell, the method comprising:
(I) a step of collecting a part of a cell population during culturing at a desired time point;
(II) a step of measuring an expression level of a predetermined gene in the collected cell population;
(III) a step of comparing the expression level of the measured gene with a threshold; and
(IV) a step of determining that when the expression level of the measured gene is equal to or higher than the threshold, the cultured cell population at the desired time point is capable of differentiating into the neural crest cell and/or the neural crest progenitor cell.

20. The method according to claim 19, wherein the predetermined gene comprises one or more genes that has enhanced expression after inducing differentiation, and/or one or more genes that has enhanced expression during an expansion culturing period.

21. The method according to claim 20, wherein the one or more genes that has enhanced expression after inducing differentiation comprise one or more selected from the group consisting of SOX10, RHOB, FOXD3, and NOTCH1, and the one or more genes that has enhanced expression during the expansion culturing period comprise one or more selected from the group consisting of SOX9, TFAP2A, NGFR, TWIST1, and EDN3.

22. The method according to any one of claims 19 to 21, wherein the desired time point is an arbitrary time point in the inducing differentiation of the pluripotent stem cell into the neural crest cell and/or the neural crest progenitor cell to obtain a cell population comprising the neural crest cell and/or the neural crest progenitor cell, or
the desired time point is an arbitrary time point in the selectively culturing a neural crest cell in the cell population comprising the neural crest cell and/or the neural crest progenitor cell.
